(19) Europäisches Patentamt
European Patent Office
Office européen des brevets

(11) Numéro de publication: **0 074 309**
**A1**

# (12) DEMANDE DE BREVET EUROPEEN

(21) Numéro de dépôt: 82401580.4

(51) Int. Cl.³: **A 61 K 49/04**

(22) Date de dépôt: 25.08.82

(30) Priorité: 28.08.81 FR 8116478

(43) Date de publication de la demande: 16.03.83 Bulletin 83/11

(84) Etats contractants désignés: BE CH DE FR GB IT LI LU NL SE

(71) Demandeur: Société dite: GUERBET S.A.
16-24 Rue Jean-Chaptal
F-93609 Aulnay-sous-Bois(FR)

(72) Inventeur: Dimo, Ioana
23 Rue du Commandant Mouchotte
F-94160 Saint Mande(FR)

(72) Inventeur: Bonnemain, Bruno
9 Rue de Reims
F-77290 Mitry Mory(FR)

(72) Inventeur: Hardouin, Michel Jean-Charles
88 Avenue Foch
F-94120 Fontenay Sous Bois(FR)

(72) Inventeur: Lautrou, Jean
21 Avenue Gambetta
F-94160 Saint Mande(FR)

(74) Mandataire: Bressand, Georges et al,
c/o CABINET LAVOIX 2 Place d'Estienne d'Orves
F-75441 Paris Cedex 09(FR)

## (54) Procédé pour augmenter la tolérance des produits opacifiants et produits opacifiants ainsi obtenus.

(57) L'invention a pour objet un procédé pour augmenter la tolérance de produits opacifiants comprenant des composés benzéniques substitués sur le noyau par plusieurs atomes d'iode, tout en obtenant une opacité du même ordre de grandeur, caractérisé en ce que l'on remplace une partie des composés benzéniques substitués sur le noyau par plusieurs atomes d'iode par des composés benzéniques substitués sur le noyau par plusieurs atomes de brome.

EP 0 074 309 A1

Croydon Printing Company Ltd

0074309

# Procédé pour augmenter la tolérance des produits opacifiants et produits opacifiants ainsi obtenus.-

La présente invention concerne des produits opacifiants utilisés pour la radiographie.

On utilise depuis longtemps comme produits opacifiants des composés iodobenzéniques présentant sur le noyau benzénique plusieurs atomes d'iode, en général 3 atomes d'iode par noyau benzénique, et divers autres substituants. Ces autres substituants sont des groupes pharmacologiquement acceptables qui permettent l'administration des composés chez l'homme et les animaux. Ces substituants sont généralement choisis pour conférer aux composés une hydrosolubilité suffisante en vue de l'administration de ces composés en solution aqueuse.

De multiples solutions ont été proposées jusqu'à présent pour augmenter la tolérance des composés iodobenzéniques utilisés comme produits opacifiants.

Un premier type de solution a consisté à synthétiser des structures présentant deux ou trois noyaux benzéniques triiodés (voir par exemple les brevets US-A-3 290 366 et GB 1 346 795).

Un second type de solution a consisté à choisir les substituants autres que les atomes d'iode de façon à obtenir une meilleure tolérance. En particulier, on s'est orienté vers des structures non ioniques, c'est-à-dire ne présentant pas de substituants ionisants tels que des groupes carboxy (voir par exemple les brevets DE-A-2 031 724 et FR-A-2 253 509).

Un troisième type de solution a consisté à synthétiser des composés di- ou tribenzéniques polyiodés dissymétriques présentant un seul groupe ionisant (voir par exemple le brevet US-A-4 014 986).

La Demanderesse a cherché à résoudre le problème de l'augmentation de la tolérance des composés iodobenzéniques d'une manière fondamentalement différente de ce qui a été fait jusqu'à présent.

Elle a découvert que, contrairement à ce que l'on pouvait attendre, si l'on remplace dans les produits opacifiants que l'on utilise en radiographie, en partie les composés iodobenzéniques par des composés bromobenzéniques, non seulement on augmente la tolérance des produits opacifiants, mais on obtient une opacité du même ordre de grandeur.

Ce dernier fait est particulièrement surprenant, car, normalement, on devait s'attendre à ce que le remplacement partiel des composés iodobenzéniques par des composés bromobenzéniques entraîne une diminution notable de l'opacité. En effet, il est connu que l'opacité d'un atome aux rayons X est sensiblement proportionnelle à la puissance 3 de son numéro atomique (J. Duheix, V. Bismuth, M. Laval-Jeantet - Traité de radiodiagnostic, vol. 1 - L'image radiologique, Masson et Cie, 1969). Le numéro atomique du brome est 35, alors que celui de l'iode est 53. L'opacité conférée par le brome devrait donc être trois à quatre fois plus faible. On pouvait donc s'attendre à ce que la présence d'atomes de brome à la place d'une partie des atomes d'iode conduise à une réduction importante de l'opacité. Au contraire, on peut obtenir par le remplacement partiel des composés iodobenzéniques par des composés bromobenzéniques des opacités qui sont du même ordre de grandeur que celles obtenues avec des produits opacifiants ne comprenant que des composés iodobenzéniques. Il s'ajoute à ceci un avantage économique important car le brome est actuellement bien moins onéreux que l'iode.

En conséquence, la présente invention a pour objet un procédé pour augmenter la tolérance de produits opacifiants comprenant des composés benzéniques substitués sur le noyau par plusieurs atomes d'iode,tout en obtenant une opacité du même ordre de grandeur, caractérisé en ce que l'on remplace une partie des composés benzéniques substitués sur le noyau par plusieurs atomes d'iode par des composés

benzéniques substitués sur le noyau par plusieurs atomes de brome.

On peut remplacer avantageusement de 1/3 à 2/3 des composés iodobenzéniques par des composés bromobenzéniques.

La présente invention a également pour objet des produits opacifiants pour la radiographie, présentant une tolérance plus grande que celle des produits opacifiants comprenant uniquement des composés iodobenzéniques et une opacité du même ordre de grandeur que ces produits opacifiants, caractérisés en ce qu'ils comprennent des mélanges de composés benzéniques substitués sur le noyau par plusieurs atomes d'iode et des composés benzéniques substitués sur le noyau par plusieurs atomes de brome.

Dans ces produits opacifiants, les composés bromobenzéniques représentent avantageusement de 1/2 à 2/1 (en moles) des composés iodobenzéniques. Dans les mélanges, les composés bromobenzéniques peuvent être des analogues des composés iodobenzéniques ou des composés de structure différente.

Le procédé selon la présente invention est tout à fait général et s'applique à tous les composés iodobenzéniques.

Les produits opacifiants selon la présente invention peuvent notamment contenir des mélanges de deux ou plusieurs des composés suivants, l'un au moins étant bromé et un autre au moins étant iodé.

I - Les composés de formule

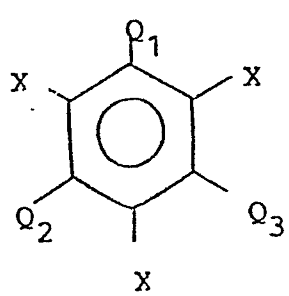

(I)

dans laquelle

les substituants X sont choisis parmi I et Br, et

$Q_1$, $Q_2$ et $Q_3$ sont des groupes pharmacologiquement acceptables.

II - Les composés de formule

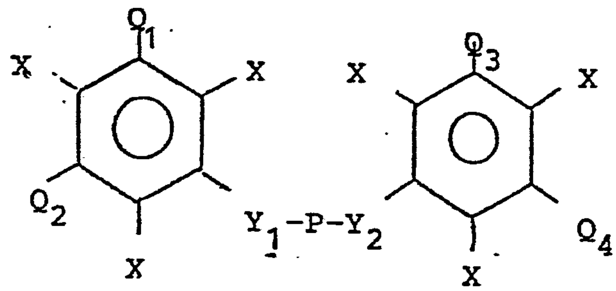

(II)

dans laquelle

les substituants X sont choisis parmi I et Br, et

$Q_1$, $Q_2$, $Q_3$, $Q_4$, $Y_1$, $Y_2$ et P sont des groupes pharmacologiquement acceptables.

III - Les composés de formule

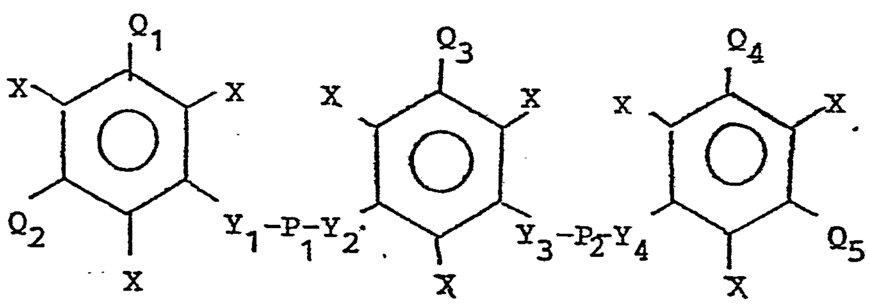

(III)

dans laquelle

les substituants X sont choisis parmi I et Br, et

$Q_1$, $Q_2$, $Q_3$, $Q_4$, $Q_5$, $Y_1$, $Y_2$, $Y_3$, $Y_4$, $P_1$ et $P_2$ sont des groupes pharmacologiquement acceptables.

IV - Les composés de formule

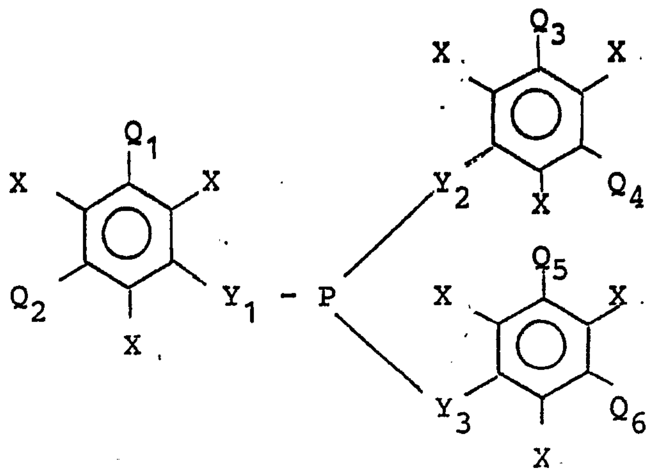

(IV)

dans laquelle

les substituants X sont choisis parmi I et Br,

et

$Q_1$, $Q_2$, $Q_3$, $Q_4$, $Q_5$, $Q_6$, $Y_1$, $Y_2$, $Y_3$ et P étant des groupes pharmaceutiquement acceptables.

On donnera ci-après des exemples de groupes de type Q, Y, Z et P.

1) <u>Groupes de type Q.</u>

a) l'hydrogène

b) des groupes aminés hydrophiles tels que

⊖ des groupes A de formule

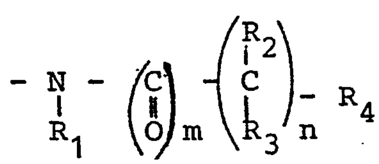

dans laquelle

m = 0, 1 ou 2

n = 0 à 6

et les radicaux $R_1$, $R_2$, $R_3$ et $R_4$ sont des groupes de formule $-(CHZ)_aT$ avec a = 0 à 5

Z = H ou OH

T = H , OH ou -COOH

$-\left[(CH_2)_b-O(CH_2)_d\right]_pH$ avec b = 1 à 5

d = 1 à 5

p = 1 à 5

- reste de sucre

Comme exemples typiques de tels groupes, on peut citer les groupes de formule :

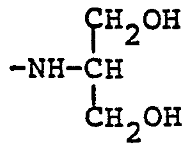 ; 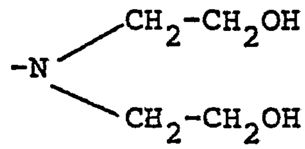 ; 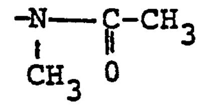 ;

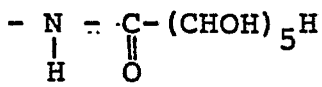 ; 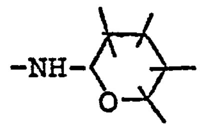

⊖ des groupes de formule

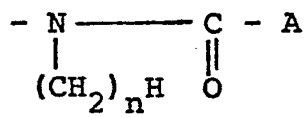

dans laquelle n = 0 à 3 et A est un groupe azoté tel que défini ci-dessus.

⊖ des groupes de formule

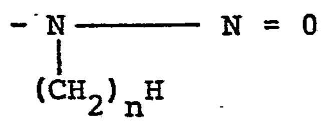

avec n = 0 à 3

⊖ des groupes de formule

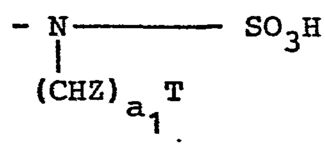

dans laquelle Z = H ou OH

T = H ou OH

$a_1$ = 0 à 3

c) des groupes éther ou ester tels que

⊖ des groupes alcoxy en $C_1$ à $C_6$ éventuellement substitués, notamment par des groupes carbonés ou des groupes aminés tels que le groupe A défini précédemment;

⊖ des groupes de formule

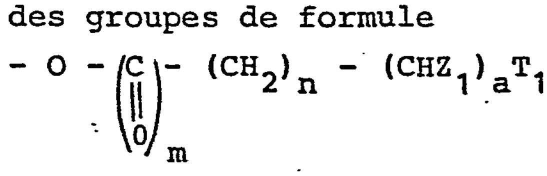

dans laquelle m = 0, 1 ou 2

n = 0 à 6

a = 0 à 6

$Z_1$ = H, OH ou $NH_2$

$T_1$ = H, OH ou $NH_2$

⊖ des groupes de formule $-O-\underset{\underset{O}{\|}}{C}-Ar$

dans laquelle Ar représente un radical aromatique

⊖ des groupes de formule

$$-O-\underset{\underset{O}{\|}}{C}-A \quad \text{ou} \quad -O-\underset{\underset{O}{\|}}{C}-\underset{\underset{O}{\|}}{C}-A$$

dans lesquelles A est tel que défini précédemment.

d) des groupes sulfonylés tels que

⊖ le groupe $-SO_3H$

⊖ les groupes de formule $-SO_2-A$ dans laquelle A est tel que défini précédemment.

e) des groupes liés par un carbone tels que

⊖ des groupes de formule $-(CHZ)_aT$

dans laquelle a = 0 à 6

Z = H ou OH

T = H ou OH

⊖ des groupes de formule $-(CH_2)_n-O-Ar$ dans laquelle Ar représente un radical aromatique

⊖ des groupes de formules

$-(CH_2)_n-\underset{\underset{O}{\|}}{C}-H$ ou 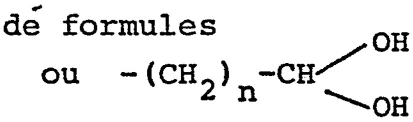

$-(CH_2)_n-\underset{\underset{O}{\|}}{C}-OH$

$-(CH_2)_n-\underset{\underset{O}{\|}}{C}-A$

dans lesquelles n = 0 à 6

A est tel que défini précédemment

⊖ des groupes de formule 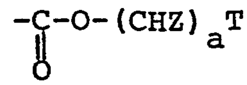

dans laquelle a = 0 à 6
Z = H ou OH
T = H ou OH

⊖ des groupes de formule 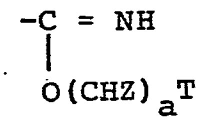

dans laquelle a = 0 à 3
Z = H ou OH
T = H ou OH

⊖ le groupe $-C \equiv N$

2) <u>Groupes de type Y</u>. (bivalents)

a) des groupes liés par un carbone tels que

⊖ des groupes de formule 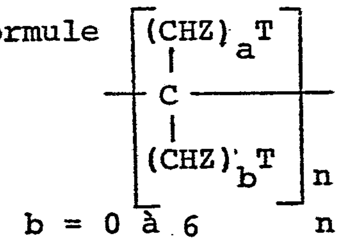

dans laquelle a, b = 0 à 6 n = 1 à 4
Z = H ou OH
T = H ou OH

⊖ le groupe 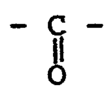

⊖ des groupes de formule 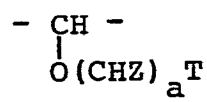

dans laquelle a = 0 à 6
Z = H ou OH
T = H ou OH

⊖ le groupe 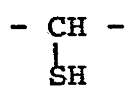

b) des groupes d'autre type tels que

⊖ le groupe 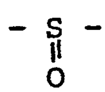

⊖ des groupes de formule

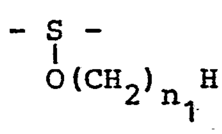 avec $n_1$ = 1 à 6

⊖ le groupe oxy - O -

⊖ des groupes de formule

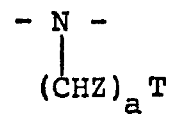

dans laquelle a = 0 à 3

Z, T = H ou OH

⊖ des groupes de formule

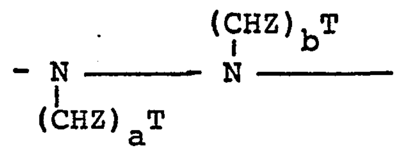

dans laquelle a, b = 0 à 3

Z, T = H ou OH

3) <u>Groupes de type P.</u>

Les groupes P divalents peuvent répondre aux formules générales

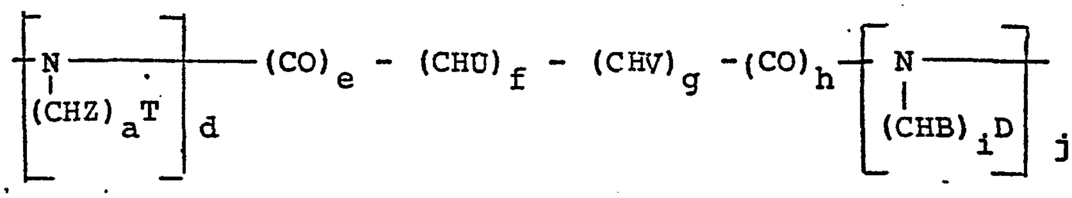

ou 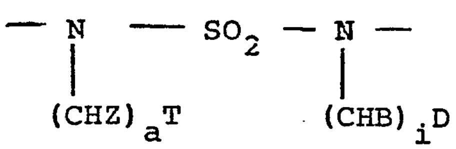

dans lesquelles

d, e, h, j = 0, 1 ou 2

a, f, g, i = 0 à 6

Z, U, B, V = H, OH, Br, $(CH_2)_nCOOH$ avec n = 0 à 3

T, D = H ou OH

D'autres restes peuvent être intercalés dans le pont, tels que les groupes de formules -O- , -S-

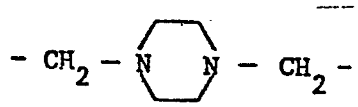

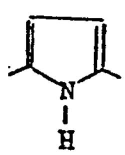 ; 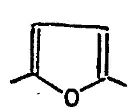 ; 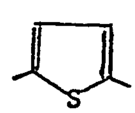

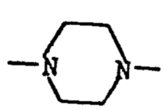 , 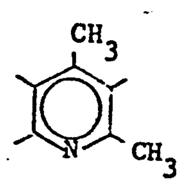

Les groupes P trivalents peuvent être des groupes de formule

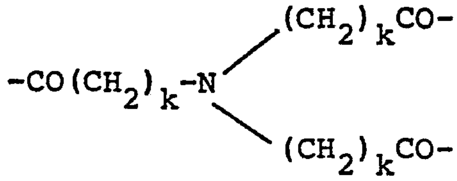

avec k = 0 à 4.

Parmi les composés bromés ou iodés de formule I, des classes particulièrement avantageuses sont celles formées par

1 - les composés de formule

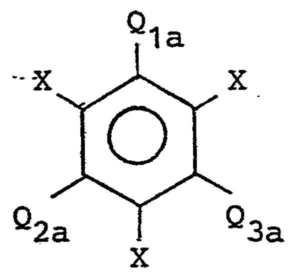

(Ia)

dans laquelle

X est Br ou I

$Q_{1a}$ est un groupe -COOH (généralement salifié par une base pharmacologiquement acceptable),

$Q_{2a}$ représente un atome d'hydrogène, un radical de formule 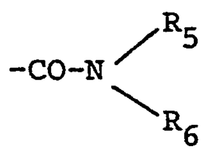, $R_5$ et $R_6$ étant un atome d'hydrogène, un radical alcoyle inférieur, hydroxyalcoyle inférieur ou alcanoyl(inf.)oxyalcoyl(inf.), un groupe amino ou un radical de formule 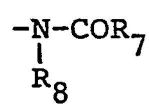, $R_7$ étant un radical alcoyle inférieur, hydroxyalcoyle inférieur ou alcoxy (inf) alcoyle (inf) et $R_8$ un atome d'hydrogène, un radical alcoyle inférieur ou hydroxyalcoyle inférieur,

$Q_{3a}$ représente un groupe amino ou un radical de formule 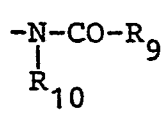 dans laquelle $R_9$ et $R_{10}$ ont les significations données respectivement pour $R_7$ et $R_8$

2 - les composés de formule

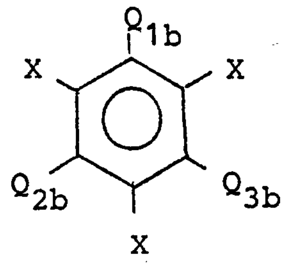

(Ib)

dans laquelle

X est Br ou I

$Q_{1b}$ représente un radical de formule

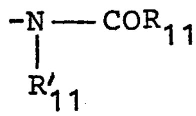

$R_{11}$ étant un radical alcoyle (inf.) polyhydroxylé

$R'_{11}$ étant un atome d'hydrogène ou un radical alcoyle inférieur

$Q_{2b}$ représente un radical de formule

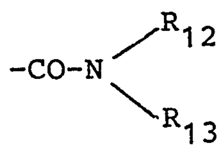 dans laquelle $R_{12}$ et $R_{13}$ ont les significations données respectivement pour $R_5$ et $R_6$

$Q_{3b}$ représente un radical de formule

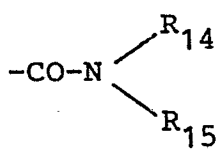 dans laquelle $R_{14}$ et $R_{15}$ ont les significations données respectivement pour $R_5$ et $R_6$ ou un radical de formule 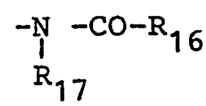 dans laquelle $R_{16}$ et $R_{17}$ ont les significations données respectivement pour $R_7$ et $R_8$.

3 - les composés de formule

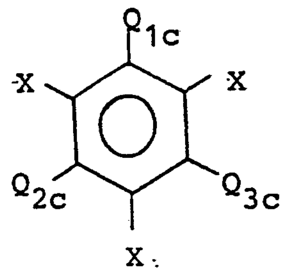

(Ic)

dans laquelle

X est Br ou I

$Q_{1c}$ représente un radical de formule $-CONH-R_{18}$ dans laquelle $R_{18}$ est un reste de sucre ou un radical alcoyle (inf.) polyhydroxylé

$Q_{2c}$ représente un radical 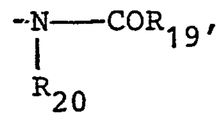, dans laquelle $R_{19}$ et $R_{20}$ ont les significations données respectivement pour $R_7$ et $R_8$, et

$Q_{3c}$ représente un radical de formule 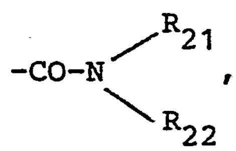, dans laquelle $R_{21}$ et $R_{22}$ ont les significations données respectivement pour $R_5$ et $R_6$, ou un radical de formule 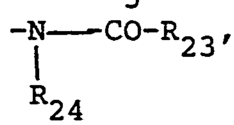, dans laquelle $R_{23}$ et $R_{24}$ ont les significations données respectivement pour $R_7$ et $R_8$.

Parmi les composés de formule II des classes particulièrement avantageuses sont celles formées par

1 - les composés de formule

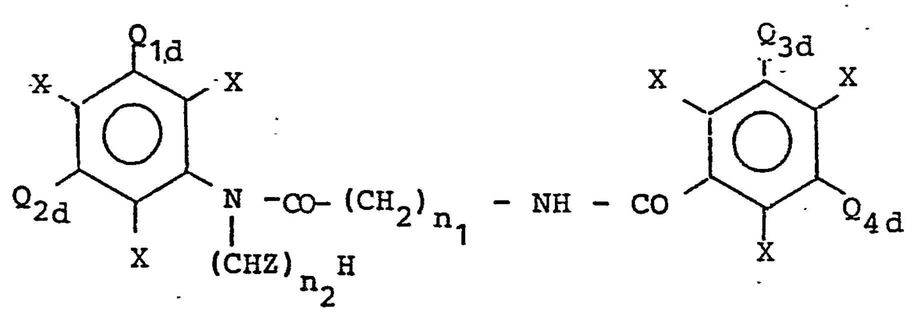

(IIa)

dans laquelle

X est Br ou I

$Q_{1d}$ est un groupe -COOH (généralement salifié

par une base pharmacologiquement acceptable).

$Q_{2d}$ et $Q_{3d}$ sont des radicaux ayant les mêmes significations que $Q_{2a}$, $Q_{2d}$ pouvant également représenter un radical $-CH_2OH$

$Q_{4d}$ représente un groupe $-NH_2$ ou un radical de formule $-\underset{R_{26}}{\underset{|}{N}}-CO-R_{25}$ dans laquelle $R_{25}$ a la signification donnée pour $R_7$ et $R_{26}$ a la signification donnée pour $R_8$ ou est un radical alcanoyle inférieur,

Z est H ou OH

$n_1$ = 1 à 5

$n_2$ = 0 à 6.

2 - Les composés de formule

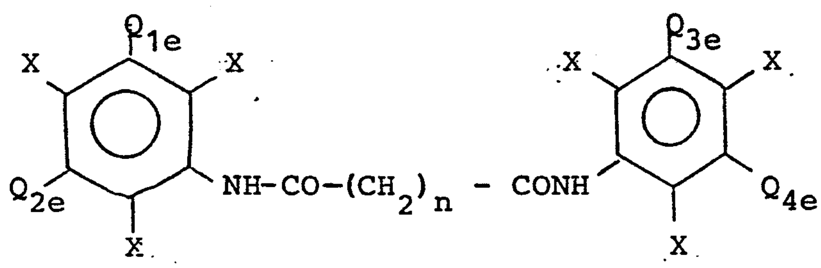

(IIb)

dans laquelle

X est Br ou I

$Q_{1e}$ et $Q_{3e}$ sont des groupes COOH (généralement salifiés par des bases pharmacologiquement acceptables)

$Q_{2e}$ et $Q_{4e}$ ont les significations données pour $Q_{2a}$.

Parmi les composés de formule IV, une classe particulièrement avantageuse est celle constituée par les composés de formule

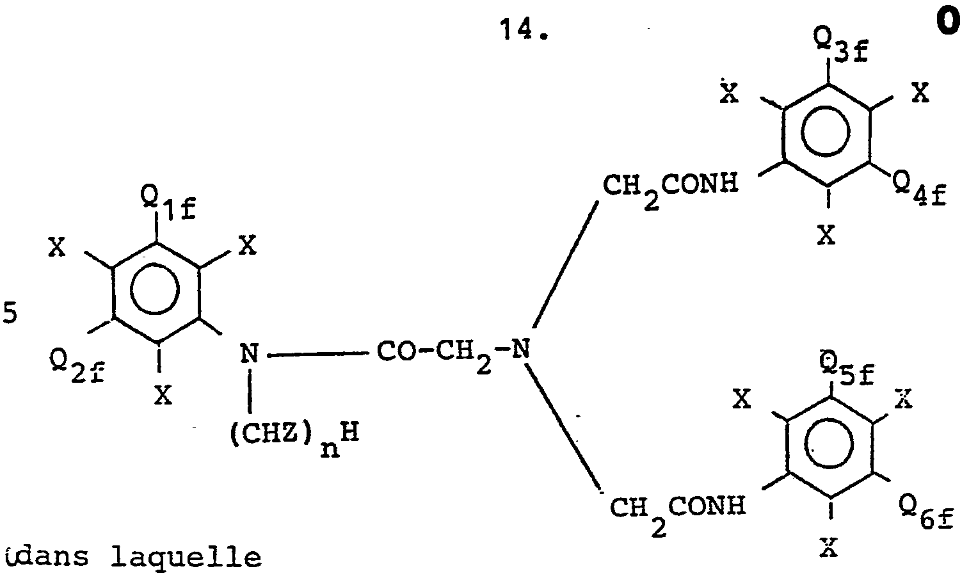

IV$_a$

dans laquelle

X est Br ou I

$Q_{1f}$ est un groupe COOH (généralement salifié par une base pharmacologiquement acceptable)

$Q_{2f}$, $Q_{3f}$, $Q_{4f}$, $Q_{5f}$ et $Q_{6f}$ ont les significations données pour $Q_{2a}$, Z est H ou OH et n = 0 à 6.

Comme autre exemple de composés de formule II on peut citer les composés de formule :

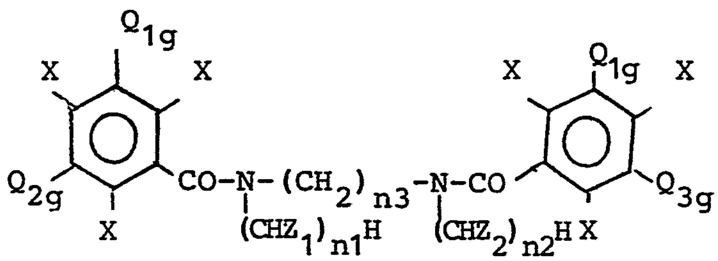

II$_c$

dans laquelle : X est Br ou I

$Q_{1g}$ représente un radical de formule

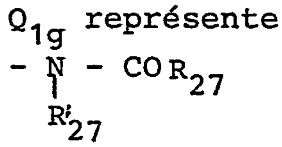

$R_{27}$ étant un radical alcoyle (inf.) polyhydroxylé

$R'_{27}$ étant un atome d'hydrogène ou un radical alcoyle inférieur,

$Q_{2g}$ et $Q_{3g}$ représentent indépendamment l'un de l'autre un radical de formule

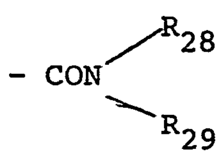

, $R_{28}$ et $R_{29}$ étant un atome d'hydrogène, un radical alcoyle inférieur, hydroxyalcoyl inférieur ou alcanoyl (inf.) oxyalcoyle (inf.), un groupe amino ou un radical de formule 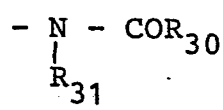

$R_{30}$ étant un radical alcoyle inf., hydroxy alcoyle inférieur ou alcoxy (inf.) alcoyle (inf.) et $R_{31}$ un atome d'hydrogène, un radical alcoyle inférieur ou hydroxyalcoyle inférieur,

$Z_1$ et $Z_2$ sont H ou OH

$n_1$ et $n_2$ = 0 à 6

$n_3$ = 0 à 4.

Comme autre exemple de composés de formule I, on peut citer les composés de formule

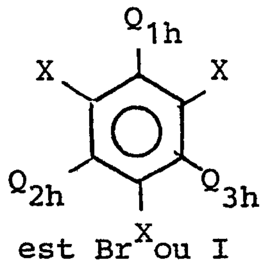

$I_d$

dans laquelle X est Br ou I

$Q_{1h}$, $Q_{2h}$ et $Q_{3h}$ représentent un groupe -COOH, un groupe -COOH salifié par une base pharmacologiquement acceptable ou un groupe de formule - 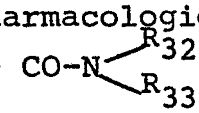

dans laquelle $R_{32}$ et $R_{33}$ représentent un atome d'hydrogène, un radical alcoyle inférieur, hydroxyalcoyle (inf.) ou alcanoyl (inf.) oxyalcoyle (inf.)

Dans les définitions ci-dessus, le terme "inférieur" appliqué aux radicaux alcoyle, alcoxy ou alcanoyle désigne généralement des radicaux ayant de 1 à 6 atomes de carbone; de plus, par radical hydroxyalcoyle, on désigne un radical alcoyle mono ou polyhydroxylé.

Les nouveaux composés bromobenzéniques utilisés dans la présente invention peuvent être préparés selon les procédés utilisés pour préparer les analogues polyiodés. A cet effet, on peut mettre en oeuvre les réactions classiques de bromation, d'alcoylation, d'acylation (par condensation d'un chlorure d'acide sur une amine ou un alcool) et de salification, largement décrites pour les analogues polyiodés.

Les composés iodobenzéniques ont été largement décrits dans la littérature et un certain nombre d'entre eux sont commercialisés.

Comme exemples de composés iodobenzéniques de formule Ia on peut citer l'acide diatrizoïque, l'acide iothalamique, l'acide métrizoïque, l'acide acétrizoïque, l'iodamide et l'acide ioxitalamique.

Comme exemple de composé iodobenzénique de formule $I_b$, on peut citer le ioglunide.

Comme exemple de composés de iodobenzéniques de formule $I_c$, on peut citer le métrizamide.

Comme exemple de composé iodobenzénique de formule $II_a$, on peut citer l'acide ioxaglique.

Comme exemples de composés iodobenzéniques de formule $II_b$, on peut citer l'acide iocarmique, l'adipiodone et l'acide ioglycamique.

Les exemples suivants illustrent la préparation des composés bromobenzéniques.

EXEMPLE I

Préparation de l'acide tribromo-2,4,6 N-méthylcarbamoyl-3 N-méthyl N-acétylamino-5 benzoïque.

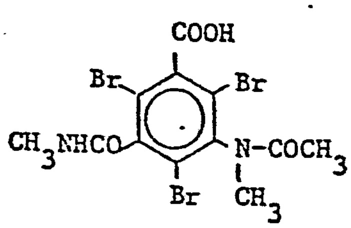

1) Préparation de l'acide tribromo-2,4,6 N-méthylcarbamoyl-3 amino-5 benzoïque.

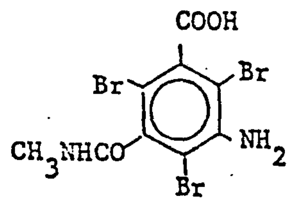

On met en suspension 194 g (1 mole) d'acide N-méthylcarbamoyl-3 amino-5 benzoïque dans 4 litres d'eau et 820 ml d'acide chlorhydrique concentré. On ajoute goutte à goutte 230 ml (9 moles) de brome. On poursuit l'agitation pendant 24 heures à température ambiante. On essore, on lave le précipité dans 2 litres d'eau à 90°C puis on sèche pendant 24 heures à 110°C.

On obtient 410 g d'acide brut, soit un rendement de 95 %.

Contrôle de pureté :

1) CCM dans l'éluant benzène/MEC/acide formique (60/25/20)

.Rf produit de départ : 0,1

.Rf produit bromé : 0,7

2) Pureté d'après le dosage de brome : 98 %.

2) Préparation de l'acide tribromo-2,4,6 N-méthylcarbamoyl-3 N-acétylamino-5 benzoïque.

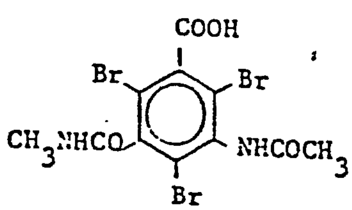

a) Condensation.

On met en suspension 230 g (0,5 mole) d'acide tribromo-2,4,6 N-méthylcarbamoyl-3 amino-5 benzoïque dans 200 ml d'anhydride acétique et 100 ml d'acide acétique. On ajoute goutte à goutte 60 ml d'acide sulfurique concentré (la température ne doit pas dépasser 55-60°C). On agite pendant 1 heure à 55°C à la fin de l'addition d'acide sulfurique. La solution obtenue est versée dans un litre d'eau + glace. Il y a précipitation. On agite à température ambiante pendant 24 heures. On essore, on lave

à l'eau. On sèche à l'étuve pendant 16 heures à 80°C.

On obtient 240 g d'acide brut, soit un rendement de 100 %.

b) Purification.

La purification se fait par cristallisation du sel d'ammonium.

On met en suspension 42 g d'acide brut dans 45 ml d'eau. On ajoute de l'ammoniaque 10N jusqu'à dissolution (soit pH = 7-8). On agite pendant 24 heures à température ambiante. Il y a cristallisation. On essore, on clairce par 10 ml d'eau. Le précipité est dissous dans 500 ml d'eau à 90°C. On effectue deux traitements au noir 3SA pendant 2 heures à 80°C. On précipite le produit par l'acide chlorhydrique 1/10. On essore, on lave à l'eau, on sèche à 80°C pendant une nuit.

On obtient 30 g de produit, soit un rendement de 71 %.

Contrôle de pureté :

1) CCM :

- dans l'éluant benzène/MEC/acide formique (60/25/20)

. Rf du produit de départ : 0,7

. Rf du produit acétylé : 0,35

- dans l'éluant butanol/acide acétique/eau (60/11/25)

. Rf du produit de départ : 0,75

. Rf du produit acétylé : 0,3

2) Pureté d'après le dosage de brome : 100 %

3) Pureté d'après le dosage à la soude : 99 %.

3) Préparation de l'acide tribromo-2,4,6 N-méthylcarbamoyl-3 N-méthyl N-acétylamino-5 benzoïque.

a) Méthylation.

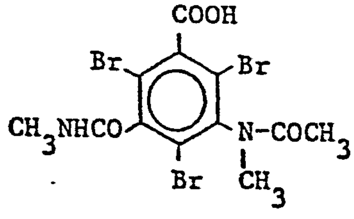

On dissout 189 g (0,4 mole) d'acide tribromo-2,4,6 N-méthylcarbamoyl-3 N-acétylamino-5 benzoïque dans 184 ml de soude 5N (0,92 mole). On ajoute 32,4 ml (0,52 mole) d'iodure de méthyle et on agite pendant 24 heures à température ambiante. La fin de réaction est contrôlée par CCM dans l'éluant : butanol/acide acétique/eau 50/11/25. On verse la solution dans 300 ml d'eau et 75 ml d'acide chlorhydrique concentré. Il y a précipitation. On laisse cristalliser pendant 5 heures, puis on essore. Le précipité est repris dans 500 ml d'eau. On ajoute de la soude 10N jusqu'à dissolution, puis on ramène le pH à 4 par addition d'acide acétique. Pour décolorer la solution on ajoute 1 ml d'une solution de bisulfite de soude. On précipite en milieu acide, on essore, on lave à l'eau et on sèche pendant 24 heures à 80°C.

On obtient 157 g de produit, soit un rendement de 81 % pour la méthylation.

b) Purification.

La purification se fait par recristallisation dans le mélange éthanol-eau.

On met en suspension 100 g d'acide brut dans 500 ml d'eau. On chauffe à 80°C, on ajoute lentement 130 ml d'éthanol à 95 %, soit jusqu'à dissolution complète. On filtre et on laisse recristalliser sous agitation pendant 24 heures. On essore, on clairce par le mélange eau-éthanol et on sèche à l'étuve pendant 24 heures à 80°C.

On obtient 62,8 g de produit que l'on dissout dans 200 ml d'eau et de soude. On ajuste à pH = 4-5 par l'acide acétique et on fait deux passages au noir. On filtre, puis on acidifie par l'acide chlorhydrique concentré. Après essorage, lavage à l'eau, séchage à 80°C pendant 24 heures, on obtient 50 g de produit pur, soit un rendement de 50 %.

Contrôle de pureté :

1) CCM dans l'éluant butanol/acide acétique/eau : 50/11/25

. Rf du produit de départ : 0,3
. Rf du produit méthylé : 0,25 et 0,35.

2) Pureté d'après le dosage au méthylate: 97 %

3) Pureté d'après le dosage de brome : 97 %.

EXEMPLE II

Préparation de l'acide tribromo-2,4,6 N-hydroxyéthylcarbamoyl-3 N-méthyl N-acétylamido-5 benzoïque

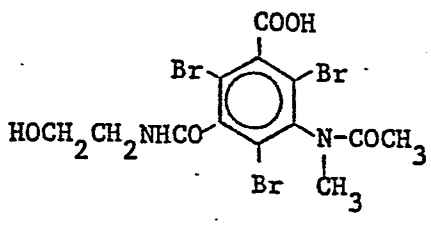

1) Préparation de l'acide tribromo-2,4,6 N-hydroxyéthylcarbamoyl-3 amino-5 benzoïque

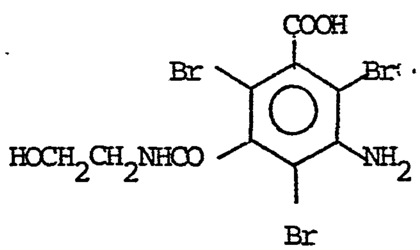

L'acide tribromo-2,4,6 N-hydroxyéthylcarbamoyl-3 amino-5 benzoïque est préparé comme dans l'exemple I-1 à partir de l'acide N-hydroxyéthylcarbamoyl-3 amino-5 benzoïque.

Rendement : 96,5 %.

Contrôle de pureté :

- CCM dans l'éluant benzène/MEC/acide formique 60/25/20

. Rf du produit de départ : 0,05

. Rf du produit bromé : 0,55

- Pureté d'après le dosage de brome : 100 %.

2) Préparation de l'acide tribromo-2,4,6 N-hydroxyéthylcarbamoyl-3 N-acétylamino-5 benzoïque

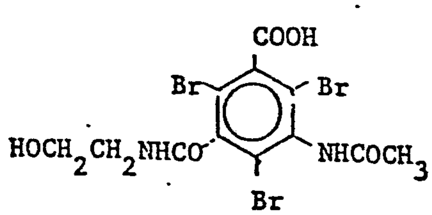

L'acide tribromo-2,4,6 N-hydroxyéthylcarbamoyl-3 N-acétylamino-5 benzoïque est préparé comme dans l'exemple I-2 à partir de l'acide tribromo-2,4,6 N-hydroxyéthylcarbamoyl-3 amino-5 benzoïque.

Rendement acétylation : 75,5 %.

Rendement purification: 65,5 %.

Contrôle de pureté :

- CCM dans l'éluant benzène/MEC/acide formique 60/25/20

. Rf produit de départ : 0,55 - Rf produit acétylé : 0,25.

- Pureté d'après le dosage de brome : 101 %
- Pureté d'après le dosage au méthylate: 100 %.

3) Préparation de l'acide tribromo-2,4,6 N-hydroxy éthylcarbamoyl-3 N-méthyl N-acétylamino-5 benzoïque

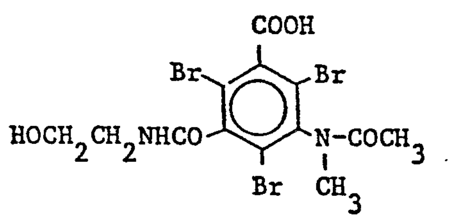

L'acide tribromo-2,4,6 N-hydroxyéthylcarbamoyl-3 N-méthyl N-acétylamino-5 benzoïque est préparé comme dans l'exemple I-3 à partir de l'acide tribromo-2,4,6 N-hydroxyéthylcarbamoyl-3 N-acétylamino-5 benzoïque.

Rendement global (de méthylation et de purification) : 20 %.

Contrôle de pureté :

- CCM dans l'éluant benzène/MEC/acide formique: 60/25/20.

. Rf du produit de départ : 0,25

. Rf du produit méthylé : 0,25

- CCM dans l'éluant butanol/$CH_3COOH$/eau 50/11/25

. Rf du produit de départ : 0,20

. Rf du produit méthylé : 0,18-0,32

- Pureté d'après le dosage au méthylate: 98 %
- Pureté d'après le dosage de brome :104 %.

EXEMPLE III

Préparation de l'acide tribromo-2,4,6 N-méthyl N-acétylamino-3 benzoïque

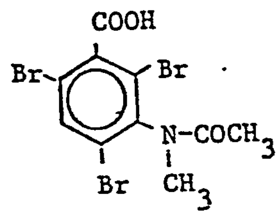

1) Préparation de l'acide tribromo-2,4,6 amino-3 benzoïque

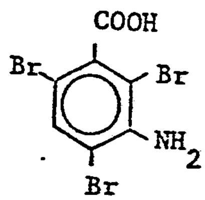

L'acide tribromo-2,4,6 amino-3 benzoïque est préparé comme dans l'exemple I-1 à partir de l'acide amino-3 benzoïque.

Rendement : 95,2 %.

Contrôle de pureté :

- CCM dans l'éluant benzène/MEC/acide formique 60/25/20 :

Rf du produit de départ : 0,2

Rf du produit bromé : 0,85

- Pureté d'après le dosage de brome : 99 %.

2) Préparation de l'acide tribromo-2,4,6 N-acétylamino-3 benzoïque

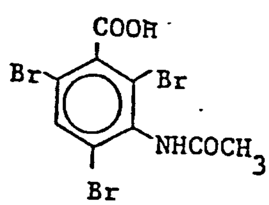

L'acide tribromo-2,4,6 N-acétylamino-3 benzoïque est préparé comme dans l'exemple I-2 à partir de l'acide tribromo-2,4,6 N-acétylamino-3 benzoïque.

Rendement : 84,5 %.

Contrôle de pureté :

- CCM dans l'éluant benzène/MEC/acide formique (60/25/20)
  . Rf du produit de départ : 0,85
  . Rf du produit acétylé : 0,6.
- Pureté d'après le dosage au méthylate de sodium : 97 %.

3) Préparation de l'acide tribromo-2,4,6 N-méthyl N-acétylamino-3 benzoïque

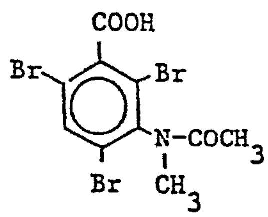

L'acide tribromo-2,4,6 N-méthyl N-acétylamino-3 benzoïque est préparé comme dans l'exemple I-3 à partir de l'acide tribromo-2,4,6 N-acétylamino-3 benzoïque.

Rendement global de méthylation et de purification : 64,5 %.

Contrôle de pureté :

- CCM dans l'éluant benzène/MEC/acide formique: 60/25/20.
  . Rf du produit de départ : 0,6
  . Rf du produit méthylé : 0,75
- CCM dans l'éluant butanol/$CH_3COOH$/eau 50/11/25
  . Rf du produit de départ : 0,45
  . Rf du produit méthylé : 0,55
- Pureté d'après le dosage au méthylate :98 %
- Pureté d'après le dosage de brome :99 %

EXEMPLE IV

Préparation de l'acide tribromo-2,4,6 amino-3 N-méthyl N-acétylamino-5 benzoïque

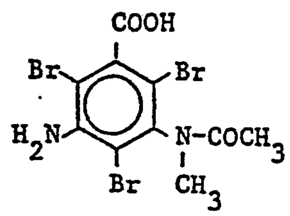

1) Préparation de l'acide tribromo-2,4,6 amino-3 N-acétylamino-5 benzoïque

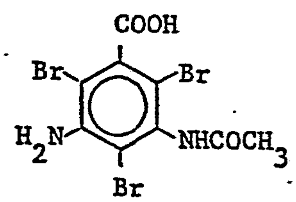

L'acide tribromo-2,4,6 amino-3 N-acétylamino-5 benzoïque est préparé comme dans l'exemple I-1 à partir de l'acide amino-3 N-acétylamino-5 benzoïque.

Rendement : 95 %.

Contrôle de pureté :

- CCM dans l'éluant benzène/MEC/acide formique 60/25/20
  . Rf du produit de départ : 0,07
  . Rf du produit bromé : 0,65
- Pureté d'après le dosage de brome : 97 %.

2) Préparation de l'acide tribromo-2,4,6 amino-3 N-méthyl N-acétylamino-5 benzoïque

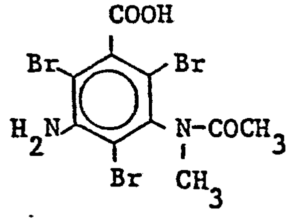

L'acide tribromo-2,4,6 amino-3 N-méthyl N-acétylamino-5 benzoïque est préparé comme dans l'exemple I-3 à partir de l'acide tribromo-2,4,6 amino-3 N-acétylamino-5 benzoïque.

Rendement : 81 %.

Contrôle de pureté :

- CCM dans l'éluant butanol/acide acétique/eau/ 50/11/25
  - . Rf du produit de départ : 0,4
  - . Rf du produit méthylé : 0,32 et 0,47
- Pureté d'après le dosage de brome : 98 %
- Pureté d'après le dosage au méthylate: 101 %.

EXEMPLE V

Préparation de l'acide adipoyldiimino-5,5'bis(tribromo-2,4,6 amino-5 N-méthyl isophtalamique)

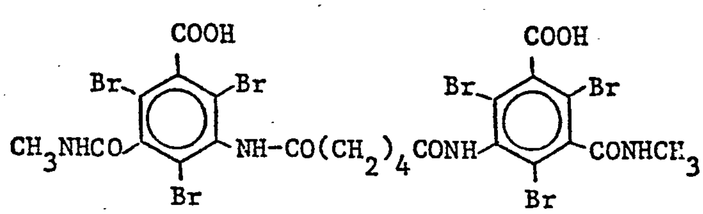

a) Condensation

On dissout 150 g (0,348 mole) d'acide tribromo-2,4,6 N-méthylcarbamoyl-3 amino-5 benzoïque dans 520 ml de diméthylacétamide. On ajoute goutte à goutte 25,5 ml (0,2 mole) de chlorure d'adipoyle en maintenant la température entre 20 et 25°C. On agite pendant 12 heures à température ambiante.

La fin de réaction est contrôlée par CCM.

On verse la solution dans 900 ml d'eau; il y a précipitation. On maintient l'agitation pendant 16 heures. Après essorage, lavage à l'eau et séchage à 60°C pendant 24 heures, on obtient 135 g de produit, soit un rendement de 80 %.

b) Purification

La purification se fait par cristallisation du sel d'ammonium.

On met en suspension 135 g de produit dans 135 ml d'eau, on ajoute de l'ammoniaque 10N jusqu'à dissolution (soit pH=7-8). On maintient sous agitation pendant 24 heures. Il y a cristallisation. Après essorage, clairçage par 20 ml d'eau, on dissout le précipité dans 500 ml d'eau et l'on fait un passage au noir 3SA. On filtre, puis on acidifie par l'acide chlorhydrique concentré. On essore, on lave à l'eau, on sèche à l'étuve pendant 16 heures à 80°C.

On obtient 58,9 g de produit purifié, soit un rendement de 43,5 % pour la purification.

Contrôle de pureté :

- CCM dans l'éluant benzène/MEC/acide formique 60/25/10
  - . Rf du produit de départ : 0,7
  - . Rf du produit condensé : 0,45
- Pureté d'après le dosage de brome :104 %
- Pureté d'après le dosage au méthylate: 93 %
- Pureté d'après le dosage au tétrabutylammonium : 104 %.

EXEMPLE VI

Préparation de l'acide adipoyldiimino-5,5'bis(tribromo-2,4,6 amino-3 benzoïque)

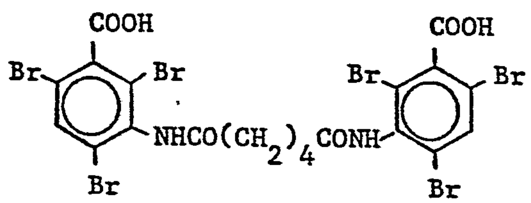

L'acide adipoyldiimino-5,5'bis(tribromo,2,4,6 amino-3 benzoïque)est préparé comme dans l'exemple V en partant de l'acide tribromo-2,4,6 amino-3 benzoïque.

Rendement de condensation : 100 %

Rendement de purification : 56 %

Contrôle de pureté :

- CCM dans l'éluant benzène/MEC/acide formique 60/25/10

. Rf du produit de départ : 0,85

. Rf du produit condensé : 0,5

- Pureté d'après le dosage de brome : 99 %

- Pureté d'après le dosage de méthylate : 101 %.

EXEMPLE VII

Préparation de l'acide tribromo-2,4,6 N-hydroxyéthylcarbamoyl-3 (tribromo-2,4,6 N-méthylcarbamoyl-3 N-méthyl N-acétylamino-5 benzoyl) glycylamino-5 benzoïque

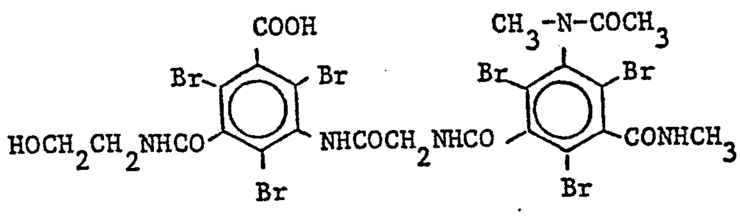

1- Préparation de l'acide tribromo-2,4,6 N-hydroxyéthyl carbamoyl-3 aminoacétamido-5 benzoïque

a) Préparation de l'acide tribromo-2,4,6 N-phtalimido acétoxyéthylcarbamoyl-3 phtalimidoacétylamino-5 benzoïque.

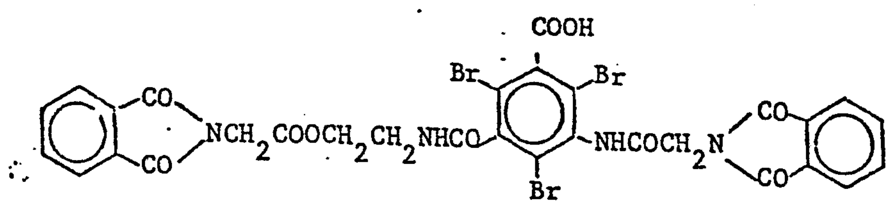

On dissout 322 g (0,7 mole) d'acide tribromo-2,4,6 N-hydroxy éthylcarbamoyl-3 amino-5 benzoïque dans 600 ml de diméthylacétamide. On ajoute par fractions 392 g (1,75 mole) de chlorure d'acide phtalyl glycine. Après 48 heures d'agitation à température ambiante, on verse la solution dans deux litres d'eau à 70°C. Il y a précipitation; on poursuit l'agitation pendant une demi-heure et on essore.

Le produit est utilisé sans séchage et sans purification dans l'étape suivante.

0074309

Contrôle :

CCM dans éluant benzène/MEC/acide formique 60/25/20 : Rf 0,7 (produit de départ : Rf 0,55).

b) Préparation de l'acide tribromo-2,4,6 N-hydroxy éthylcarbamoyl-3 aminoacétamido-5 benzoïque.

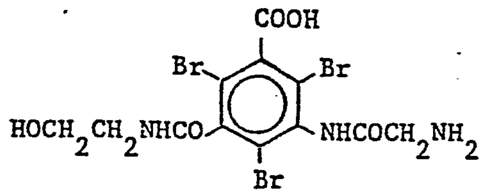

On met en suspension le produit obtenu précédemment dans 2,4 l d'eau et 204 ml d'hydrate d'hydrazine. On chauffe à 90°C pendant 1 heure, on maintient l'agitation pendant 48 heures à température ambiante . Il y a cristallisation. Ensuite, on essore et on claircе à l'eau. On obtient un produit qui contient 10 à 15 % de phtalhydrazide. Le produit est repris dans 1 litre d'eau et 100 ml d'acide sulfurique concentré; le mélange est porté à 90°C. On sépare l'insoluble par filtration, puis on ajuste le pH à 3-4 par l'ammoniaque. On laisse la cristallisation se poursuivre pendant une nuit à température ambiante.

Après essorage, lavage à l'eau, séchage à l'étuve, on récupère 175 g de produit, soit un rendement de 48,5%.

Contrôle de pureté :

1) CCM dans éluant benzène/MEC/acide formique 60/25/20 : Rf 0,05 (tache jaune orangé par révélation à la ninhydrine).

Il reste environ 1 % de phtalhydrazide à Rf 0,75.

2) Pureté d'après le dosage de brome : 98 %.

2-Préparation du chlorure d'acide tribromo-2,4,6 N-méthylcarbamoyl-3 N-méthyl N-acétylamino-5 benzoïque

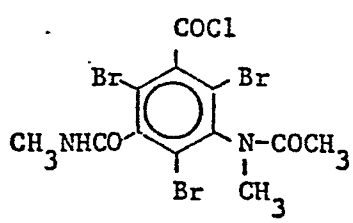

On met en suspension 296 g (0,59 mole) d'acide tribromo-2,4,6 N-méthylcarbamoyl-3 N-méthyl N-acétylamino-5 benzoïque dans 600 ml de chlorure de thionyle. On chauffe à 80°C avec agitation pendant 3 heures. On obtient une solution; on évapore sous vide le chlorure de thionyle en excès. Le résidu pâteux est repris dans 500 ml d'éther isopropylique; on agite à température ambiante pendant 24 heures. Il y a cristallisation. Après essorage, claircage par l'éther isopropylique, on lave le précipité sous agitation pendant 24 heures à température ambiante dans 250 ml d'acétone. On essore, on clairce par 50 ml d'acétone, puis on sèche le produit sous vide.

On obtient 150 g de produit beige clair, soit un rendement de 50,5 %.

Contrôle de pureté :

1) CCM (après réaction avec la monoéthanolamine, en excès dans le diméthylacétamide) dans éluant benzène/méthyléthylcétone/acide formique 60/25/20) :

. Rf du produit de départ 0,57

. Rf du produit condensé avec la monoéthanolamine 0,35

2) Dosage de chlorure d'acide par la propylamine : 105 %.

3 - Préparation de l'acide tribromo-2,4,6 N-hydroxyéthylcarbamoyl-3 (tribromo-2,4,6 N-méthylcarbamoyl-3 N-méthyl N-acétylamino-5 benzoyl) glycylamino-5 benzoïque

a) Condensation

On met en suspension 135 g (0,296 mole) d'acide tribromo-2,4,6 N-hydroxyéthylcarbamoyl-3 N-aminoacétamido-5 benzoïque dans un mélange de diméthylacétamide (300 ml) et de triéthylamine (107 ml, soit 0,74 mole). On ajoute à cette suspension 150 g, soit 0,296 mole, de chlorure d'acide tribromo-2,4,6 N-méthyl carbamoyl-3 N-méthyl N-acétylamino-5 benzoïque. On agite à 45°C pendant 3 heures. La fin de réaction est contrôlée par CCM : il reste moins de 3 % de produit de départ. On verse la solution dans 1 litre d'eau, puis on acidifie

par l'acide chlorhydrique concentré. Il se forme un léger précipité; on maintient l'agitation à température ambiante pendant 8 jours.

Après essorage, lavage à l'eau, séchage à 60°C pendant 24 heures, on obtient 68,2 g de produit, soit un rendement de 24 %.

b) Purification

On dissout 68,2 g de produit dans 130 ml d'éthanol absolu au reflux. Puis, on laisse recristalliser à température ambiante pendant 24 heures. On essore, on clairce. On dissout le produit dans 200 ml d'eau et de soude. On ajuste à pH=4-5 par l'acide acétique et l'on fait deux passages au noir. On filtre, puis on acidifie par l'acide chlorhydrique concentré.

Après essorage, lavage à l'eau, séchage à 60°C pendant 24 heures, on obtient 43 g de produit, soit un rendement de 63 % pour la purification.

Contrôle de pureté

- CCM dans l'éluant benzène/MEC/acide formique 60/25/20

. Rf de l'amine : 0,07

. Rf de l'acide correspondant au chlorure d'acide : 0,7

. Rf du produit condensé : 0,4

- Pureté d'après le dosage de brome : 100 %

- Pureté d'après le dosage au méthylate : 97 %.

EXEMPLE VIII

Préparation de l'acide tribromo-2,4,6 N-hydroxyéthylcarbamoyl-3 (tribromo-2,4,6 N-hydroxyéthylcarbamoyl-3 N-méthyl N-acétylamino-5 benzoyl) glycylamino-5 benzoïque

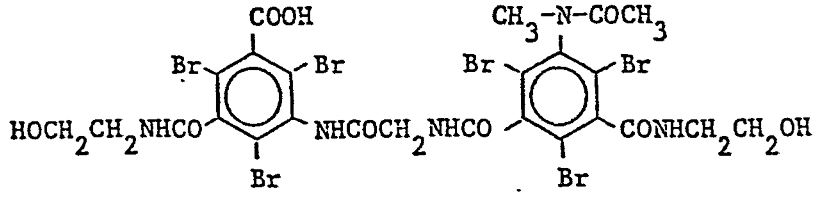

1) Préparation de l'acide tribromo-2,4,6 N-acétoxyéthylcarbamoyl-3 N-méthyl N-acétylamino-5 benzoïque

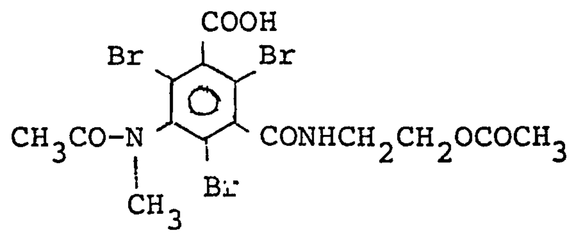

On met en suspension 270 g (0,52 mole) d'acide tribromo-2,4,6 N-hydroxyéthylcarbamoyl-3 N-méthyl N-acétylamino-5 benzoïque dans 1,3 litre de dioxanne. On ajoute 78 ml (1,04 mole) de chlorure d'acétyle, puis on chauffe à 80°C pendant 8 heures. On élimine le dioxanne par évaporation sous vide. On obtient 290 g de produit, soit un rendement de 98,6 %.

Ce produit est utilisé sans purification.

Contrôle de pureté :

- CCM dans l'éluant benzène/MEC/acide formique 60/25/20

. Rf du produit de départ : 0,25

. Rf du produit O-acétylé : 0,40.

2) Préparation du chlorure d'acide tribromo-2,4,6 N-acétoxyéthyl carbamoyl-3 N-méthyl N-acétylamino-5 benzoïque

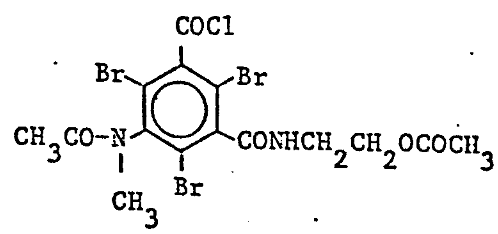

On met en suspension 290 g (0,52 mole) d'acide tribromo-2,4,6 N-acétoxyéthylcarbamoyl-3 N-méthyl N-acétylamino-5 benzoïque dans 500 ml de chlorure de thionyle. On chauffe à 80°C pendant 4 heures. On élimine sous vide l'excès de chlorure de thionyle.

Après séchage, on obtient 300 grammes de produit, soit un rendement de 100 %. Ce produit est utilisé sans purification.

3) Condensation

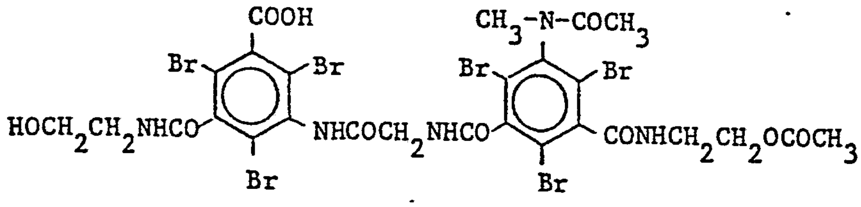

La préparation de l'acide tribromo-2,4,6 N-hydroxyéthylcarbamoyl-3 (tribromo-2,4,6 N-acétoxyéthylcarbamoyl-3 N-méthyl N-acétylamino-5 benzoyl) glycylamino-5 benzoïque est effectuée comme dans l'exemple VII, à partir de l'acide tribromo-2,4,6 N-hydroxyéthylcarbamoyl-3 aminoacétamido-5 benzoïque et du chlorure d'acide tribromo-2,4,6 N-acétoxy éthylcarbamoyl-3 N-méthyl N-acétylamino-5 benzoïque.

Rendement de condensation : 62,5 %.

Ce produit est utilisé sans purification.

4) Saponification

On dissout 146 g (0,138 mole) d'acide tribromo-2,4,6 N-hydroxyéthylcarbamoyl-3 (tribromo-2,4,6 N-acétoxy éthylcarbamoyl-3 N-méthyl N-acétylamino-5 benzoyl) glycylamino-5 benzoïque dans 270 ml de soude 2N. On chauffe à 45°C pendant 2 heures. On ajuste à pH=6-7 par l'acide chlorhydrique et l'on fait deux passages au noir. On filtre, puis on précipite en milieu acide (pH=1). On essore, on clairce et l'on sèche à l'étuve pendant 20 heures à 60°C. On obtient 54 g de produit, soit un rendement de 24 %.

Contrôle de pureté

- CCM dans l'éluant benzène/MEC/acide formique 60/25/20

. Rf du produit de départ : 0,1

. Rf du produit saponifié : 0,05.

5) Purification

Elle se fait par cristallisation dans l'isopropanol.

On met en suspension 54 g de produit brut dans 54 ml d'isopropanol. On chauffe à 80°C pendant 2 heures. On agite à température ambiante pendant une nuit; il y a cristallisation. On essore, on clairce par l'isopropanol,

puis on lave le produit à l'eau. Après séchage à l'étuve à 80°C pendant 16 heures, on obtient 21 g de produit, soit un rendement de purification de 34 %.

Contrôle de pureté :

- CCM :
  - dans l'éluant benzène/MEC/acide formique 60/25/20 : Rf 0,05
  - dans l'éluant butanol/acide acétique/eau 50/11/25 : Rf 0,2 et 0,3.

EXEMPLE IX

Préparation de l'acide tribromo-2,4,6 N-méthylcarbamoyl-3 (tribromo-2,4,6 N-méthyl N-acétylamino-3 benzoyl) glycyl-amino-5 benzoïque

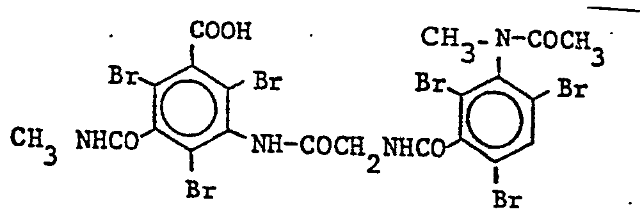

1) Préparation de l'acide tribromo-2,4,6 N-méthyl carbamoyl-3 aminoacétylamino-5 benzoïque

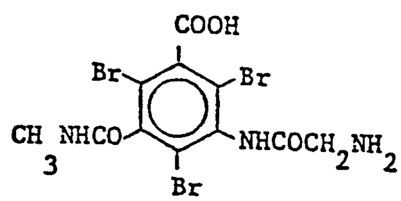

Il est préparé comme à l'exemple VII-1 à partir de l'acide tribromo-2,4,6 N-méthyl carbamoyl-3 amino-5 benzoïque.

Rendement obtenu : 45 %

Contrôle de pureté :

- CCM
  - dans éluant benzène/MEC/acide formique 60/25/20 : Rf 0,1
  - dans éluant butanol/acide acétique/eau 50/11/25 : Rf 0,07
- Pureté d'après le dosage de brome : 96 %.

2) Préparation du chlorure d'acide tribromo-2,4,6 N-méthyl N-acétylamino-3 benzoïque

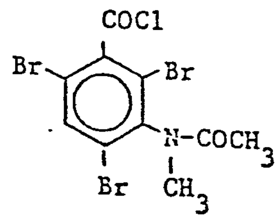

Il est préparé comme à l'exemple VII-2 en utilisant l'acide tribromo-2,4,6 N-méthyl N-acétyl amino-3 benzoïque.

Rendement obtenu : 85 %

Contrôle de pureté :

- CCM (après réaction avec la monoéthanolamine, en excès dans le diméthylacétamide) dans éluant benzène/méthyléthylcétone/acide formique 60/25/20

. Rf du produit de départ : 0,7

. Rf du produit condensé avec la monoéthanolamine : 0,5

3) Préparation de l'acide tribromo-2,4,6 N-méthyl carbamoyl-3 (tribromo-2,4,6 N-méthyl N-acétylamino-3 benzoyl) glycylamino-5 benzoïque

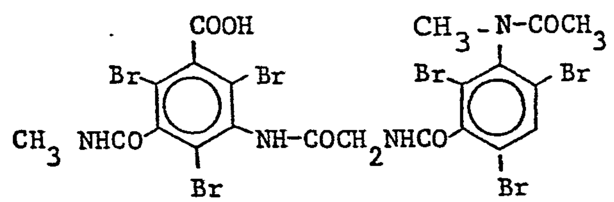

L'acide tribromo-2,4,6 N-méthylcarbamoyl-3 (tribromo-2,4,6 N-méthyl N-acétylamino-3 benzoyl) glycylamino-5 benzoïque est préparé comme dans l'exemple VII en partant de l'acide tribromo-2,4,6 N-méthylcarbamoyl-3 aminoacétamido-5 benzoïque et du chlorure d'acide tribromo-2,4,6 N-méthyl N-acétylamino-3 benzoïque.

Rendement de condensation : 85 %

Rendement de purification : 13,5 %.

Contrôle de pureté : CCM dans l'éluant benzène/MEC/acide formique 60/25/20

Rf de l'amine : 0,05

Rf de l'acide correspondant au chlorure d'acide : 0,85

Rf du produit condensé : 0,5

- Pureté d'après le dosage du brome : 96 %
- Pureté d'après le dosage au méthylate : 103 %.

EXEMPLE X

Préparation de l'acide tribromo-2,4,6 N-méthylcarbamoyl-3bis /(tribromo-2,4,6 bis(N-hydroxyéthylcarbamoyl)-3,5 phényl) carbamoyl méthyl/ amino acétamido-5 benzoïque

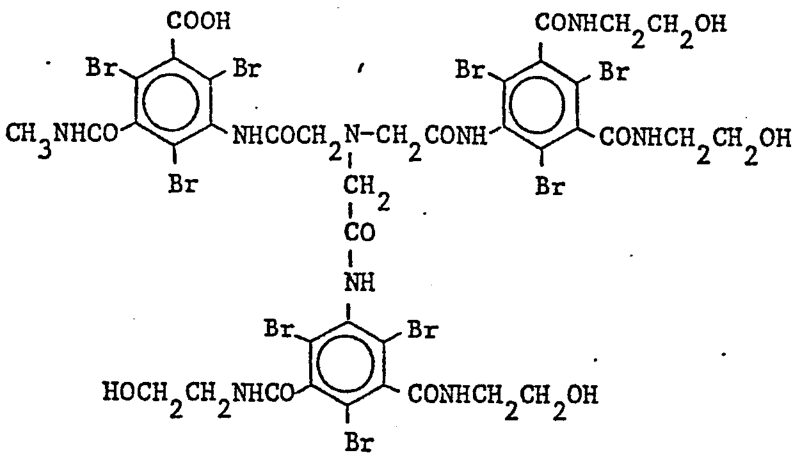

1) Préparation de la tribromo-2,4,6 bis(hydroxyéthyl carbamoyl)-3,5 chloracétanilide

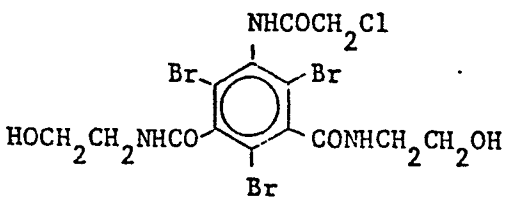

a) Préparation de l'acide tribromo-2,4,6 amino-5 isophtalique

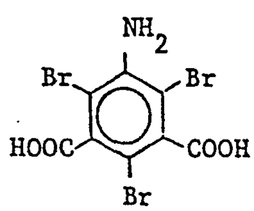

Cet acide est préparé comme dans l'exemple I-1 à partir de l'acide amino-3 isophtalique.

Rendement : 86,5 %.

Contrôle de pureté :

- CCM dans l'éluant benzène/MEC/acide formique 60/25/20

. Rf du produit de départ : 0,1

. Rf du produit bromé : 0,8

- Pureté d'après le dosage de brome : 99 %.

b) Préparation du chlorure d'acide tribromo-2,4,6 amino-5 isophtalique

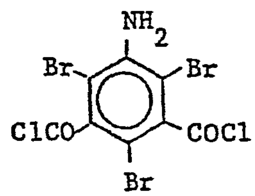

On met en suspension 729 g (1,73 mole) d'acide tribromo-2,4,6 amino-5 isophtalique dans 1800 ml de chlorure de thionyle. On chauffe à 80°C pendant 8 heures. On élimine sous vide le chlorure de thionyle en excès. On reprend le concentrat dans 1 litre d'éther isopropylique. Il y a cristallisation. On essore, on clairce par l'éther isopropylique. Après séchage sous vide, on obtient 465 g, soit un rendement de 60 %.

Contrôle de pureté :

- CCM dans l'éluant benzène/MEC/acide formique 60/25/20

. Rf de l'acide de départ : 0,8

. Rf du produit après condensation avec la monoéthanolamine : 0,4.

- Pureté d'après le dosage de chlorure d'acide par la propylamine : 105 %.

c) Préparation de la tribromo-2,4,6 bis(hydroxyéthylcarbamoyl)-3,5 aniline.

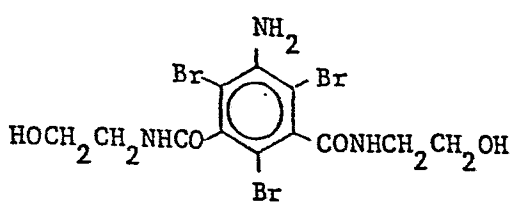

On dissout 184 g (0,404 mole) de chlorure d'acide tribromo-2,4,6 amino-5 isophtalique dans 180 ml de

diméthylacétamide. On ajoute goutte à goutte 145 ml (2,4 moles) de monoéthanolamine en maintenant la température entre 20 et 25°C. On agite pendant 16 heures à température ambiante. On verse dans un litre d'eau glacée. Il y a précipitation. On agite pendant 24 heures à température ambiante, on essore, on lave à l'eau et on sèche à 80°C pendant 16 heures.

On obtient 168 g de produit, soit un rendement de 84 %.

Contrôle de pureté :

- CCM dans l'éluant benzène/MEC/acide formique 60/25/20 : Rf 0,4.
- Pureté d'après le dosage de brome: 98 %.

d) Préparation de la tribromo-2,4,6 bis(hydroxyéthylcarbamoyl)-3,5 chloracétanilide.

- Condensation

On dissout 515 g (1,02 mole) du produit obtenu en (c) dans 775 ml de diméthylacétamide. On ajoute goutte à goutte 377 ml (4,7 moles) de chlorure de chloracétyle en maintenant la température entre 20 et 30°C. On agite pendant 16 heures à température ambiante, on verse dans 4,5 l d'eau glacée. Il y a précipitation. On essore, on clairce à l'eau. Ce produit est utilisé sans séchage.

Contrôle de pureté :

- CCM dans l'éluant benzène/MEC/acide formique 60/25/20
  - . Rf du produit de départ : 0,4
  - . Rf du produit condensé : 0,85

- Saponification

On met en suspension le produit ci-dessus dans 1750 ml de soude 2N. On agite à température ambiante pendant 16 heures. On acidifie par l'acide chlorhydrique (pH=1). On essore, on lave à l'eau et l'on sèche à 80°C pendant 16 heures. On obtient 215 g, soit un rendement de 36 %.

Contrôle de pureté :

- CCM dans benzène/MEC/acide formique 60/25/20

. Rf du produit de départ : 0,4

. Rf du produit chloracétylé: 0,2

- Pureté d'après le dosage de brome : 99 %
- Pureté d'après le dosage de chlore : 105 %.

2) Préparation de l'acide tribromo-2,4,6 N-méthylcarbamoyl-3 bis /[(tribromo-2,4,6 bis (N-hydroxyéthylcarbamoyl)-3,5 phényl) carbamoyl méthyl]/ amino acétamido-5 benzoïque

a) Condensation

On mélange une solution de 10 g (0,02 mole) d'acide tribromo-2,4,6 N-méthylcarbamoyl-3 amino acétamido-5 benzoïque dans 18 ml de soude normale avec la solution de 23,2 g (0,04 mole) de tribromo-2,4,6 bis (hydroxyéthylcarbamoyl)-3,5 chloracétanilide dans 40 ml de soude normale.

Après une heure de chauffage à 85°C, on rajoute 18 ml de soude normale, puis on maintient le chauffage à 85°C pendant 20 heures. On refroidit la solution à 20°C. On acidifie à pH=1 par l'acide chlorhydrique concentré. Il se forme une gomme qui cristallise après 16 heures à température ambiante. On essore, on lave à l'eau et l'on sèche à l'étuve à 60°C pendant 24 heures. On obtient 29 g d'acide brut.

b) Purification

On met en suspension les 29 g d'acide brut dans 60 ml d'eau et l'on ajuste à pH=7 par la soude. On filtre l'insoluble puis on acidifie à pH inférieur ou égal à 1, par l'acide chlorhydrique concentré. On essore le précipité et on lave à l'eau.

Le précipité est mis en suspension dans 10 ml d'eau, on ajoute de l'ammoniaque jusqu'à neutralité. Dans la solution, on ajoute 2 g de chlorure d'ammonium. On agite à température ambiante pendant 48 heures. Il y a cristallisation. On essore, on dissout le précipité dans 50 ml d'eau. On traite deux fois au noir. On précipite par l'acide chlorhydrique. Après essorage, lavage à l'eau et séchage, on obtient 5,2 g de produit purifié.

Contrôle de pureté

- CCM dans l'éluant butanol/acide acétique/ eau 50/11/25
  . Rf du produit chloré de départ : 0,65
  . Rf du produit aminé de départ : 0,07
  . Rf du produit de condensation : 0,2
- Pureté d'après le dosage de brome : 98,7 %
- P u r e t é d ' a p r è s l e dosage au méthylate de sodium : 95 %.

EXEMPLE XI

Préparation de la tribromo-2,4,6 (N-méthyl acétamido)-3 (N-hydroxyéthylcarbamoyl)-5 N-gluconyl aniline

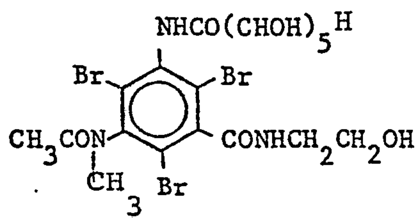

1) Préparation du chlorure d'acide tribromo-2,4,6 N-méthyl N-acétyl amino-3 amino-5 benzoïque

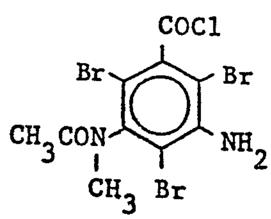

On met en suspension 200 g (soit 0,446 mole) d'acide tribromo-2,4,6 N-méthyl N-acétyl amino-3 amino-5 benzoïque dans 200 ml d'éther isopropylique. On ajoute goutte à goutte 200 ml de chlorure de thionyle. On agite pendant 4 heures à 80°C et pendant une nuit à température ambiante. On évapore le mélange à l'évaporateur rotatif jusqu'à obtention d'un résidu et l'on reprend ce résidu dans 400 ml d'éther isopropylique. On agite à température ambiante pendant 24 heures; il y a cristallisation. Après essorage, clairçage et séchage à 40°C pendant 24 heures,

on obtient 165 g de produit, soit un rendement de 79,3 %.

Contrôle de pureté

- CCM dans l'éluant benzène/MEC/acide formique 60/25/20

. Rf du produit de départ : 0,7-0,8

. Rf du chlorure d'acide : 0,5 (après condensation avec la monoéthanolamine)

- Pureté d'après le dosage à la propylamine: 90,5 %.

2) Préparation de la tribromo-2,4,6 N-hydroxyéthylcarbamoyl-3 N-méthyl N-acétylamino-5 aniline

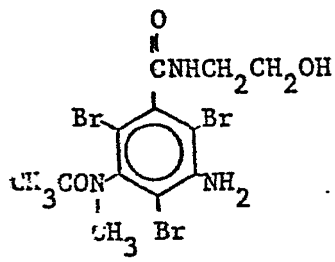

On dissout 65,3 g (0,140 mole) de chlorure d'acide tribromo-2,4,6 N-méthyl N-acétyl amino-3 amino-5 benzoïque dans 100 ml de diméthylacétamide. On ajoute goutte à goutte 25,2 ml d'éthanolamine (0,42 mole), en refroidissant éventuellement de façon que la température ne dépasse pas 25°C. On agite pendant deux jours à température ambiante.

On verse la solution dans un mélange de 400 ml d'eau et 80 g de glace. Il y a précipitation. On maintient l'agitation pendant 48 heures. Après essorage, lavage, clairçage et séchage, on obtient 56,4 g de produit, soit un rendement de 82,5 %.

Contrôle de pureté:

- CCM dans l'éluant benzène/MEC/acide formique 60/25/20

. Rf du produit de départ : 0,7-0,8

. Rf du produit condensé : 0,55.

3) Préparation de la tribromo-2,4,6 N-acétoxyéthylcarbamoyl-3 N-méthyl N-acétylamino-5 aniline

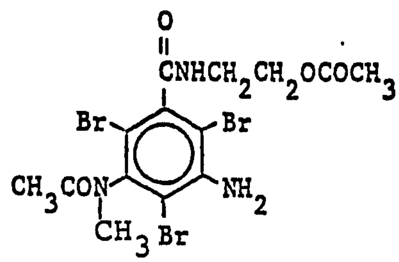

On met en suspension 56,4 g (0,115 mole) de tribromo-2,4,6 N-hydroxyéthylcarbamoyl-3 N-méthyl N-acétylamino-5 aniline dans un mélange de 74,8 ml d'acide acétique et 0,78 ml d'acide sulfurique. On agite pendant 3 heures à 80°C puis à température ambiante pendant 12 heures. On verse la solution dans 260 ml d'eau permutée et quelques glaçons; il y a cristallisation. Après clairçage et séchage on obtient 46 g de produit, soit un rendement de 75,4 %.

Contrôle de pureté :

- CCM dans l'éluant benzène/MEC/acide formique 60/25/20
  . Rf de l'amine OH : 0,55
  . Rf de l'amine O-acétylée: 0,75.

4) Préparation de la tribromo-2,4,6 (N-méthyl acétamido)-3 (N-acétoxyéthylcarbamoyl)-5 N-pentaacétoxy gluconyl aniline

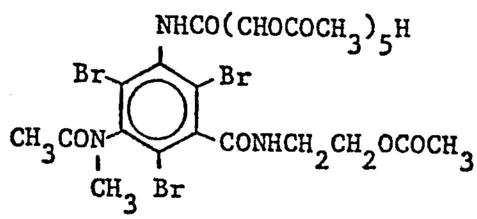

On met en suspension 28 g (0,0573 mole) de tribromo-2,4,6 N-acétoxyéthylcarbamoyl-3 N-méthyl N-acétylamino-5 aniline dans 36 ml de diméthylacétamide et l'on ajoute par fractions, 36,5 g de chlorure d'acide gluconique pentaacétylé (0,086 mole). On agite à température ambiante pendant 48 heures. On ajoute 78 ml d'eau dans la solution; on agite pendant 30 minutes. On extrait la

phase aqueuse au dichloroéthane par 4 fois 150 ml. On lave la phase organique par 4 fois 250 ml d'une solution de bicarbonate de sodium à 5 %, puis par 2 fois 500 ml d'eau.

Après séchage sur chlorure de calcium, on filtre et on évapore sous vide le dichloroéthane. On obtient 30,5 g de produit, soit un rendement de 63 %.

Contrôle de pureté :

- CCM dans l'éluant butanol/acide acétique/eau 50/11/25

. Rf de l'amine OH : 0,2

. Rf de l'amine O-acétylée : 0,65

. Rf du produit condensé avant saponification: 0,5.

5) Préparation de la tribromo-2,4,6 (N-méthylacétamido)-3 (N-hydroxyéthylcarbamoyl)-5 N-gluconyl aniline

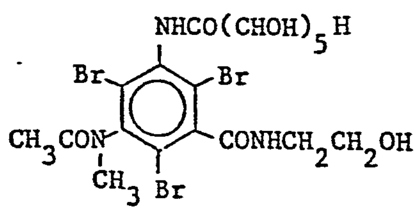

On dissout 30,5 g (0,033 mole) de tribromo-2,4, 6 (N-méthylacétamido)-3 (N-acétoxyéthylcarbamoyl)-5 N-pentaacétoxy gluconyl aniline dans un mélange de 83 ml d'eau, 13 ml d'hydrate d'hydrazine et 15 ml de méthanol. On agite à 45°C pendant 6 heures et à température ambiante pendant 12 heures. On introduit dans le mélange 40 ml d'eau et 36 g d'anhydride phtalique. On agite à 70°C pendant 4 heures et à température ambiante pendant 48 heures.

Après essorage, on traite au noir 3SA les jus de filtration à 60°C pendant 3 heures, on évapore les jus au tiers du volume initial, on extrait par 6 fois 200 ml d'acétate d'éthyle.

Après évaporation à sec, on obtient 13 g de produit, soit un rendement de 60 %.

Contrôle de pureté :

- CCM dans l'éluant benzène/MEC/acide formique 60/25/10
  - . Rf du produit avant saponification : 0,05
  - . Rf du produit après saponification : 0,55

EXEMPLES XII et XIII.

En opérant comme aux exemples VII et VIII, on a obtenu les composés suivants :

| Ex. | Formule | Rf éluant 1* | Rf éluant 2* |
|---|---|---|---|
| XII | 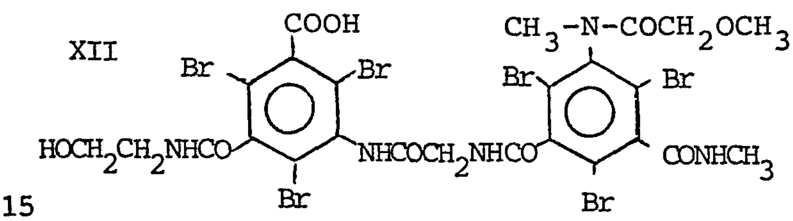 | 0,1 | 0,25 |
| XIII | 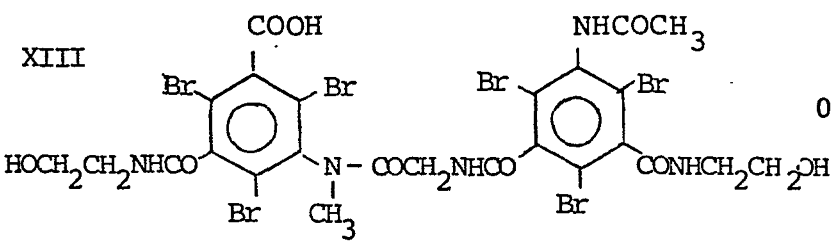 | 0, 05 | 0,2 et 0,25 |

*Eluant 1 : benzène/méthyléthylcétone/acide formique (60/25/20)

*Eluant 2 : n-butanol/acide acétique/eau (50/11/25)

EXEMPLE XIV.

Préparation du N,N'-bis(tribromo-2,4,6 N-méthyl acétamido-3 N-gluconylamino-5 benzoyl)diamino-1,2-éthane.

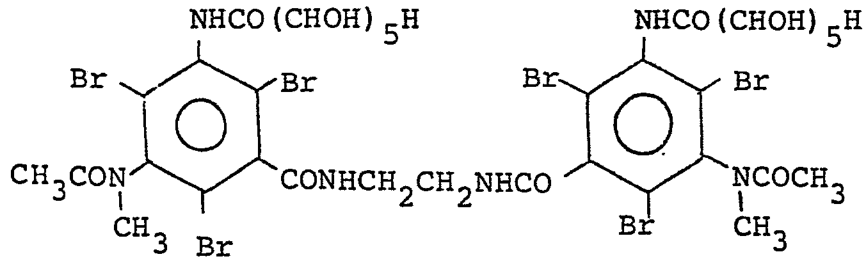

a) Préparation du chlorure d'acide tribromo-2,4,6 N-méthyl acétamido-3 amino-5 benzoïque.

20 g de l'acide correspondant dissous dans 40 ml de $SOCl_2$ sont chauffés 2 heures à 80°. Après évaporation du chlorure de thionyle, l'huile est précipitée dans 100 ml d'éther isopropylique. Après agitation 1 heure, essorage, lavage à l'acétate d'éthyle, essorage, séchage, on obtient 17 g du composé, soit un rendement de : 81,5 %.

b) Préparation du N,N'-bis(tribromo-2,4,6 N-méthyl acétamido-3 amino-5 benzoyl)diamino-1,2-éthane.

A 100 g de chlorure d'acide obtenu en a) (0,215 mole) dissous dans 190 ml de DMAC, on ajoute 10 ml (0,15 mole) d'éthylène diamine et 30 ml de triéthylamine. Après agitation à température ambiante pendant 4 jours, l'insoluble est essoré, le filtrat est précipité par 950 ml d'$H_2O$. Après essorage, lavage, séchage, on obtient 85,3 g de produit, soit 44 % de rendement.

CCM benzène/MEC/acide formique (50/20/30)

chlorure d'acide + éthanolamine Rf: 0,70-0,74

produit obtenu Rf: 0,90.

c) Préparation du N,N'-bis(tribromo-2,4,6 N-méthyl acétamido-3 N-pentaacétoxy gluconylamino-5 benzoyl) diamino-1,2-éthane.

On dissout 0,089 mole du composé obtenu ci-dessus dans 180 ml de DMAC. On ajoute 0,445 mole de chlorure d'acide gluconique pentaacétylé en poudre, qui se dissout au fur et à mesure.

Après agitation 20 heures à température ambiante, le mélange est précipité par 1,3 litre d'eau glacée. On maintient l'agitation une nuit. Après essorage, le produit brut est repris par le dichloroéthane. La phase organique lavée avec $NaHCO_3$ 5 % puis avec de l'eau est évaporée. L'huile cristallise dans l'éther de pétrole.

Rendement : 40 %.

CCM benzène/MEC/acide formique Rf 0,38 (60/25/20) (produit de départ 0,46)

d) Déprotection.

La déprotection est effectuée par du méthylate de sodium/méthanol.

CCM butanol/$H_2O$/acide acétique (50/25/20) Rf 0,34-0,40.

EXEMPLE XV

Préparation de l'acide tribromo-2,4,6 benzène tricarboxylique-1,3,5 et de ses dérivés.

a) Préparation de l'acide tribromo-2,4,6 cyano-5 isophtalique.

On dissout 232 g (0,555 mole) d'acide tribromo-2,4,6 amino-5 isophtalique dans 500 ml d'acide sulfurique concentré puis refroidit à l'aide d'un bain de glace la solution à une température comprise entre 0 et 5°C. On ajoute alors peu à peu en 1 heure 77 g (1,11 mole) de nitrite de sodium en maintenant la température à 0-5°C, puis agite encore 2 h à cette température. Le mélange est ensuite versé lentement sur de la glace sous agitation et l'excès de nitrite de sodium est détruit par une quantité suffisante d'urée en solution. Toujours en respectant la température de 0-5°C on neutralise la suspension à pH : 6 par addition de soude 5N. Le mélange est alors additionné à une solution de 69 g (0,694 mole) de chlorure cuivreux et de 89 g (1,82 mole) de cyanure de sodium dans

1 l d'eau et la solution est agitée une nuit à température ambiante. On amène le pH de la solution à 3 par addition d'acide chlorhydrique, essore le précipité de sels de cuivre, puis amène le pH à 1 par de l'acide chlorhydrique. On refroidit la solution au bain de glace et laisse sous agitation pendant 16 heures. On essore le précipité obtenu, le reprend par 400 ml d'eau sous agitation plus une quantité suffisante de soude pour dissolution et pH neutre. On agite la solution une heure avec du noir 3 SA pour décolorer, filtre le noir, précipite par de l'acide chlorhydrique dilué. On obtient après essorage, lavage par un peu d'eau 130 g de produit blanc (Rendement: 55 %).

Par recristallisation dans 400 ml d'éthanol on obtient 77,5 g de produit purifié. Point de fusion supérieur à 300°C.

b) Préparation de l'acide tribromo-2,4,6 carbamoyl-5 isophtalique 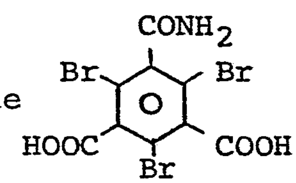

On dissout 60 g (0,140 mole) d'acide tribromo-2,4,6 cyano-5 isophtalique dans 120 ml (0,6 mole) de soude 5N. On agite ensuite la solution 3h à 50°C, puis on ajoute à température ambiante 50 cm³ d'acide chlorhydrique concentré. On refroidit par un bain de glace et agite pendant 2 h. Après essorage du précipité, lavage par un peu d'eau glacée on obtient après séchage 60 g de produit blanc (Rendement : 96 %). Point de fusion supérieur à 300°C.

CCM: benzène/MEC/acide formique 60/25/20 Rf: 0,3.

c) Préparation de l'acide tribromo-2,4,6 benzène tricarboxylique-1,3,5 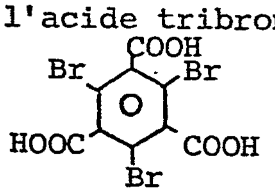

L'acide tribromo-2,4,6 benzène tricarboxylique-1,3,5 peut être obtenu à partir de l'acide tribromo-2,4,6 carbamoyl-5 isophtalique par l'action d'un agent de nitrosation qui peut être le nitrate de sodium dans l'acide sulfurique ou l'acide chlorhydrique, un alkylnitrite ou le tétrafluoroborate de nitrosonium dans un solvant organique. 52,5 g (0,123 mole) d'acide tribromo-2,4,6 carbamoyl-5 isophtalique sont mis en suspension dans un mélange de 700 ml d'eau et 700 ml d'acide chlorhydrique concentré. On chauffe la suspension à 90°C et ajoute peu à peu en 7 h une solution de 41 g (0,595 mole) de nitrite de sodium dans 700 ml d'eau. On agite encore 1 h après l'addition puis évapore la solution obtenue à sec. On obtient 90 g de résidu que l'on reprend par 500 ml d'éther sous agitation pendant 2 h. On élimine le chlorure de sodium insoluble par filtration. La phase éthérée est évaporée à sec et l'on obtient 49 g de poudre blanche (Rendement: 93 %). Point de fusion supérieur à 300°C.

CCM: Benzène/MEC/Acide formique 60/25/20 Rf: 0,4.

d) Préparation du trichlorure de l'acide tribromo-2,4,6 benzène tricarboxylique-1,3,5.

On met en suspension 45 g (0,1 mole) d'acide tribromo-2,4,6 benzène tricarboxylique-1,3,5 dans 80 ml (1,1 mole) de chlorure de thionyle. On ajoute 2 ml de diméthylformamide et agite 2 h à reflux. On laisse refroidir. Un solide blanc cristallise. On l'essore, lave avec un peu de cyclohexane. On obtient après séchage 43 g de produit sous forme d'aiguilles blanches. (Rendement: 85 %). Point de fusion: 202°C.

e) Préparation du tribromo-2,4,6 tris(dihydroxypropyl-2,3 carbamoyl)-1,3,5 benzène.

25 g (0,05 mole) de trichlorure d'acide tribromo-2,4,6 benzène tricarboxylique-1,3,5 sont dissous dans 50 $cm^3$ de diméthylacétamide. On ajoute ensuite

peu à peu une solution de 30 g (0,33 mole) d'amino propane diol-2,3 dans 60 ml de diméthylacétamide. On agite ensuite le mélange réactionnel pendant 3 h à 50°C puis après avoir amené le mélange à pH neutre par addition d'acide chlorhydrique on l'évapore à sec. Le résidu est repris par un minimum d'eau et extrait au phénol. Après traitement de la phase phénolée par l'éther éthylique et extraction à l'eau, on évapore la phase aqueuse sous vide. On obtient 25 g de produit blanc. (Rendement: 76 %).

CCM : Benzène/MEC/Acide formique 60/25/20 Rf: 0,05
Isobutanol/Isopropanol/Ammoniaque 30/30/40 Rf: 0,55 à 0,60

f) Préparation du tribromo-2,4,6 tris(N-méthyl pentahydroxyhexyl-2,3,4,5,6 carbamoyl)-1,3,5 benzène.

Préparé de la même façon que pour le produit ci-dessus (e).

CCM : Benzène/MEC/Acide formique 60/25/20 Rf: 0,05
Isobutanol/Isopropanol/Ammoniaque 30/30/40 Rf: 0,10 et 0,15

g) Préparation du tribromo-2,4,6 tris(bis-hydroxyéthyl carbamoyl)-1,3,5 benzène.

Préparé de la même façon qu'en (e).

CCM : Benzène/MEC/Acide formique 60/25/20 Rf: 0,05
Isobutanol/Isopropanol/Ammoniaque 30/30/40 Rf: 0,45.

On donnera ci-après des résultats d'essais comparatifs effectués avec les mélanges de composés bromobenzéniques et de composés iodobenzéniques.

1 - Opacité

L'opacité a été mesurée indirectement à l'aide de l'impression produite sur un film photographique par un faisceau de rayons X traversant une cuve contenant une solution aqueuse des composés. La transparence du film après développement a été mesurée à l'aide d'un densitomètre optique. Dans chaque cas, la mesure a été effectuée sur le même film et avec la même source de rayons X (70 kV) par comparaison avec un produit opacifiant servant de référence.

Comme produit de référence, on a utilisé l'Hexabrix qui est une solution de sels de sodium et de méthylglucamine de l'acide ioxaglique à 32 % en iode.

Les mélanges ont été testés sous forme de solutions aqueuses de mélanges de sels de méthylglucamine des composés iodés et bromés contenant au total le même nombre de moles de composés que l'Hexabrix.

Les résultats sont donnés dans le tableau I ci-après

TABLEAU I

| Produit opacifiant | Opacité relative/Hexabrix |
|---|---|
| Mélange équimolaire de sel de méthylglucamine d'acide ioxaglique et d'analogue bromé (Exemple VII) | 0,905 |

On constate que l'opacité du mélange est très proche de celle de l'Hexabrix. Pour obtenir avec les mélanges la même opacité qu'avec une mole de produit uniquement iodé, il faut seulement 0,55 mole de produit iodé et 0,55 mole de produit bromé.

Par ailleurs, pour tenir compte du fait que les produits de contraste sont dilués dans l'organisme, on a effectué des mesures d'opacité en opérant différentes dilutions. On a constaté que la perte d'opacité pouvait être très faible. Ainsi, pour un mélange en solution aqueuse comprenant 40 % en poids de Telebrix 38 (contenant 38 % d'iode) et 60 % en poids d'analogue bromé (composé de l'exemple II-2), la perte d'opacité par rapport au Telebrix 38 varie de 2,5 à 9,5 % suivant les dilutions.

Pour un mélange comprenant 50 % en poids de Contrix et 50 % en poids d'analogue bromé (composé de l'exemple I-2), la perte d'opacité par rapport au Contrix varie de 8,1 à 12 % suivant les dilutions.

Pour un mélange comprenant 50 % en poids de Me-Contrix et 50 % en poids d'analogue bromé (composé de l'exemple I-3) la perte d'opacité par rapport au Me-Contrix varie de 6 à 7,9 % suivant les dilutions.

2 - Toxicité aigüe intracisternale

La toxicité aigüe intracisternale a été mesurée selon la méthode de E. Melartin, P. Tuohimaa, R. Dabb, Investigative Radiology, 1970, vol. 5, n° 1, 13-21.

Les résultats sont donnés dans le tableau II.

TABLEAU II

| Produit opacifiant | Toxicité aigüe IC (DL50) | |
|---|---|---|
| | Rat mole/rat | Souris g Eq iode/kg |
| Contrix (iothalamate de méthylaglucamine) | 0,028 | |
| Mélange équimolaire Contrix + analogue bromé (sel de méthylglucamine du composé de l'exemple I-2) | 0,033 | |
| Telebrix | | 0,2 |
| Mélange équimolaire Telebrix + analogue bromé (sel de méthylglucamine du composé de l'exemple II-2) | | 0,35 |

3- Liaison protéique

Le degré d'interaction avec les protéines est un indice de la tolérance des produits opacifiants. Plus cette interaction est faible et meilleure est la tolérance.

La liaison protéique a été étudiée selon la technique Brodersen (J. of Clinical Investigation, 1974, vol. 4, 1353-1364).

Les résultats sont donnés dans le Tableau III.

TABLEAU III

| Produit opacifiant | Liaison protéique $moles^{-1}$ |
|---|---|
| Ioxaglâte de méthyl-glucamine | 122 |
| Mélange équimolaire ioxa-glate de méthylglucamine + analogue bromé (sel de méthylglucamine du composé de l'exemple VII) | 102 |
| Adipiodone de méthyl-glucamine | 12800 |
| Mélange équimolaire adipio-done de méthylglucamine + analogue bromé (sel de méthylglucamine du composé de l'exemple VI) | 8100 |

Les mélanges de composés iodés et de composés bromés peuvent donc être utilisés comme produits opacifiants à des fins radiologiques.

La forme pharmaceutique préférée est constituée par des solutions aqueuses de mélanges de composés iodés et bromés.

Les solutions aqueuses contiennent généralement de 5 à 100 g de mélange de composés iodés et de composés bromés et la quantité injectable de telles solutions peut varier généralement de 5 à 1000 ml.

On donnera ci-après des exemples de compositions selon la présente invention.

Composition A

| | |
|---|---|
| Iothalamate de méthylglucamine | 30 g |
| Sel de méthylglucamine du composé de l'exemple I-2 | 25 g |
| Eau pour préparation injectable Q.S.P. | 100 ml |

Composition B

| | |
|---|---|
| Ioxitalamate de méthylglucamine | 24 g |
| Sel de méthylglucamine du composé de l'exemple II-2 | 30 g |
| Eau pour préparation injectable Q.S.P. | 100 ml |

Composition C

| | |
|---|---|
| Ioxaglate de méthylglucamine | 30 g |
| Sel de sodium du composé de l'exemple I-3 | 25 g |
| Eau pour préparation injectable Q.S.P. | 100 ml |

Composition D

| | |
|---|---|
| Ioxaglate de méthylglucamine | 25 g |
| Sel de méthylglucamine du composé de l'exemple VII | 30 g |
| Eau pour préparation injectable Q.S.P. | 100 ml |

Composition E

| | |
|---|---|
| Ioxaglate de méthylglucamine | 30 g |
| Sel de méthylglucamine du composé de l'exemple I-2 | 25 g |
| Eau pour préparation injectable Q.S.P. | 100 ml |

REVENDICATIONS

1. Procédé pour augmenter la tolérance de produits opacifiants comprenant des composés benzéniques substitués sur le noyau par plusieurs atomes d'iode, tout en obtenant une opacité du même ordre de grandeur, caractérisé en ce que l'on remplace une partie des composés benzéniques substitués sur le noyau par plusieurs atomes d'iode par des composés benzéniques substitués sur le noyau par plusieurs atomes de brome.

2. Procédé selon la revendication 1, caractérisé en ce que l'on remplace de 1/3 à 2/3 des composés iodobenzéniques par des composés bromobenzéniques.

3. Produits opacifiants pour la radiographie, présentant une tolérance plus grande que celle des produits opacifiants comprenant uniquement des composés iodobenzéniques, et une opacité du même ordre de grandeur que ces produits opacifiants, caractérisés en ce qu'ils comprennent des mélanges de composés benzéniques substitués sur le noyau par plusieurs atomes d'iode et des composés benzéniques substitués sur le noyau par plusieurs atomes de brome.

4. Produits opacifiants selon la revendication 3, caractérisés en ce que les composés bromobenzéniques représentent de 1/2 à 2/1 (en moles) des composés iodobenzéniques.

5. Produits opacifiants selon la revendication 3, caractérisés en ce qu'ils contiennent des mélanges de deux ou plusieurs composés suivants, l'un au moins étant bromé et un autre au moins étant iodé,

I - Les composés de formule

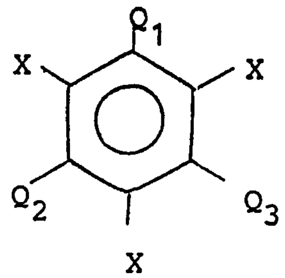

(I)

dans laquelle

les substituants X sont choisis parmi I et Br, et

$Q_1$, $Q_2$ et $Q_3$ sont des groupes pharmacologiquement acceptables,

II - Les composés de formule

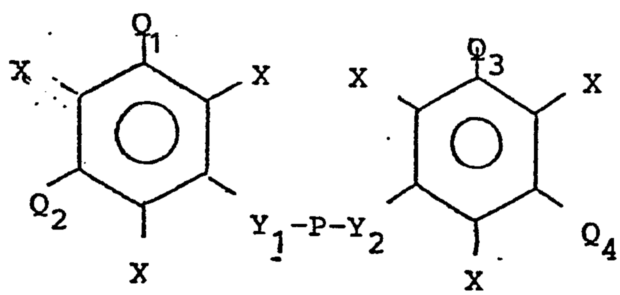

(II)

dans laquelle

les substituants X sont choisis parmi I et Br, et

$Q_1$, $Q_2$, $Q_3$, $Q_4$, $Y_1$, $Y_2$ et P sont des groupes pharmacologiquement acceptables,

III - Les composés de formule

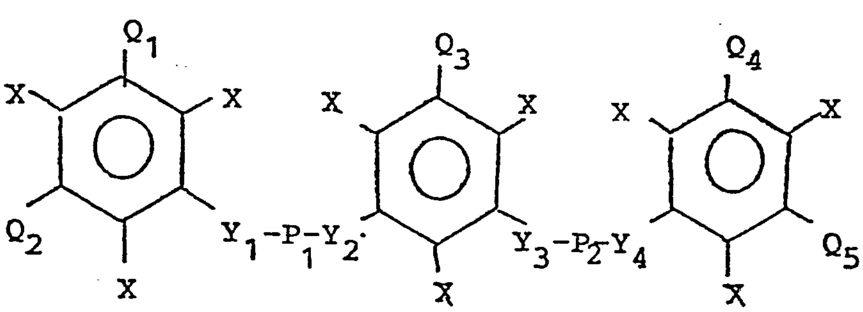

(III)

dans laquelle

les substituants X sont choisis parmi I et Br, et

$Q_1$, $Q_2$, $Q_3$, $Q_4$, $Q_5$, $Y_1$, $Y_2$, $Y_3$, $Y_4$, $P_1$ et $P_2$ sont des groupes pharmacologiquement acceptables,

IV - Les composés de formule

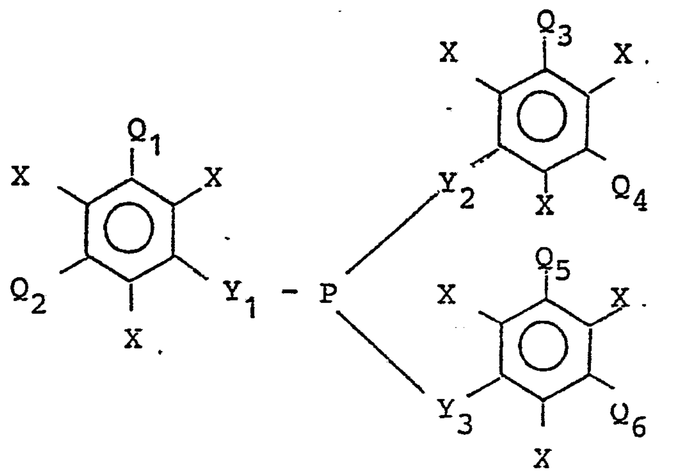

(IV)

dans laquelle

les substituants X sont choisis parmi I et Br, et

$Q_1$, $Q_2$, $Q_3$, $Q_4$, $Q_5$, $Q_6$, $Y_1$, $Y_2$, $Y_3$ et P étant des groupes pharmaceutiquement acceptables.

6 . Produits opacifiants selon la revendication 3, caractérisés en ce qu'ils contiennent des mélanges de deux ou plusieurs composés suivants, l'un au moins étant bromé et un autre au moins étant iodé :

1 - les composés de formule

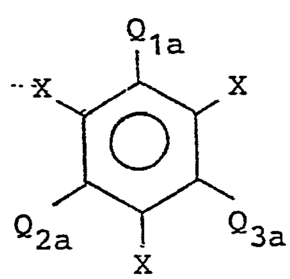

(Ia)

dans laquelle

X est Br ou I

$Q_{1a}$ est un groupe -COOH (généralement salifié par une base pharmacologiquement acceptable),

$Q_{2a}$ représente un atome d'hydrogène, un radi-

cal de formule 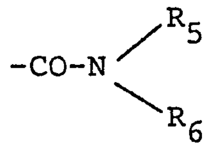 , $R_5$ et $R_6$ étant un atome d'hydrogène, un radical alcoyle inférieur, hydroxyalcoyle inférieur ou alcanoyl (inf.)oxyalcoyl(inf.) un groupe amino ou un radical de formule 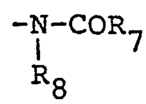 , $R_7$ étant un radical alcoyle inférieur, hydroxyalcoyle inférieur ou alcoxy (inf) alcoyle (inf) et $R_8$ un atome d'hydrogène, un radical alcoyle inférieur ou hydroxyalcoyle inférieur,

$Q_{3a}$ représente un groupe amino ou un radical de formule 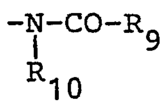 dans laquelle $R_9$ et $R_{10}$ ont les significations données respectivement pour $R_7$ et $R_8$

2 - les composés de formule

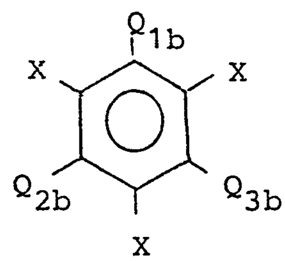

(Ib)

dans laquelle

X est Br ou I

$Q_{1b}$ représente un radical de formule

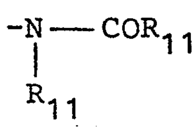

$R_{11}$ étant un radical alcoyle(inf.) polyhydroxylé

$R'_{11}$ étant un atome d'hydrogène ou un radical alcoyle inférieur

$Q_{2b}$ représente un radical de formule

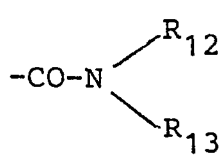 dans laquelle $R_{12}$ et $R_{13}$ ont les significations données respectivement pour $R_5$ et $R_6$

$Q_{3b}$ représente un radical de formule

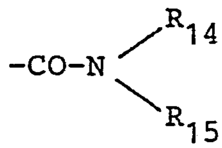 dans laquelle $R_{14}$ et $R_{15}$ ont les significations données respectivement pour $R_5$ et $R_6$ ou un radical de formule 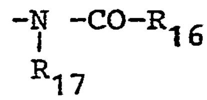 dans laquelle $R_{16}$ et $R_{17}$ ont les significations données respectivement pour $R_7$ et $R_8$.

3 - les composés de formule

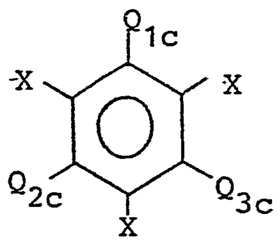

(Ic)

dans laquelle X est Br ou I

$Q_{1c}$ représente un radical de formule $-CONH-R_{18}$ dans laquelle $R_{18}$ est un reste de sucre ou un radical alcoyle (inf.) polyhydroxylé

$Q_{2c}$ représente un radical 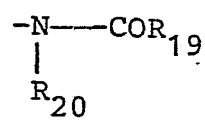, dans laquelle $R_{19}$ et $R_{20}$ ont les significations données respectivement pour $R_7$ et $R_8$, et

$Q_{3c}$ représente un radical de formule

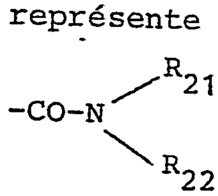, dans laquelle $R_{21}$ et $R_{22}$ ont les significations données respectivement pour $R_5$ et $R_6$, ou un radical de formule 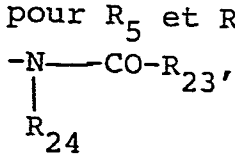, dans laquelle $R_{23}$ et $R_{24}$ ont les significations données respectivement pour $R_7$ et $R_8$.

4 - les composés de formule

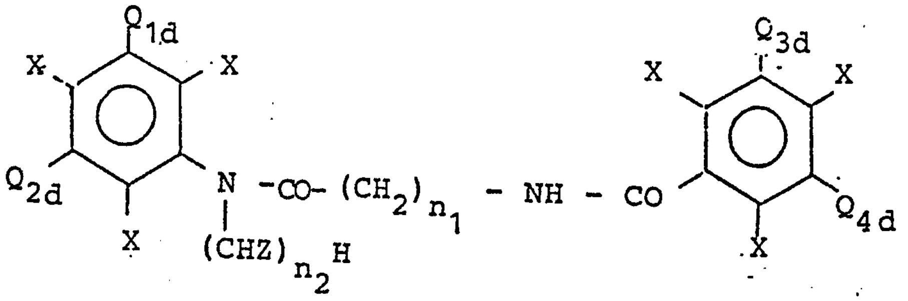

(IIa)

dans laquelle

X est Br ou I

$Q_{1d}$ est un groupe -COOH (généralement salifié par une base pharmacologiquement acceptable).

$Q_{2d}$ et $Q_{3d}$ sont des radicaux ayant les mêmes significations que $Q_{2a}$, $Q_{2d}$ pouvant également représenter un radical $-CH_2OH$

$Q_{4d}$ représente un groupe $-NH_2$ ou un radical de formule $-\underset{\displaystyle R_{26}}{\underset{|}{N}}-CO-R_{25}$ dans laquelle $R_{25}$ a la signification donnée pour $R_7$ et $R_{26}$ a la signification donnée pour $R_8$ ou est un radical alcanoyle inférieur,

Z est H ou OH

$n_1$ = 1 à 5

$n_2$ = 0 à 6.

5 - Les composés de formule

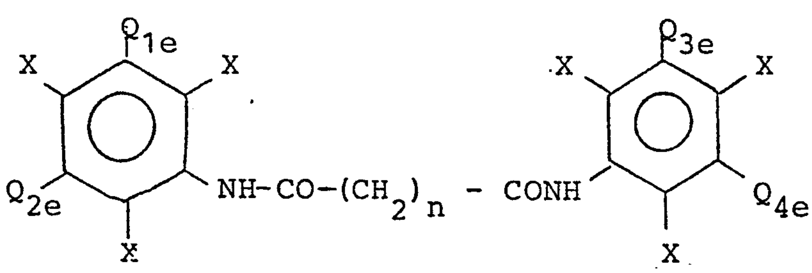

(IIb)

dans laquelle

X est Br ou I

$Q_{1e}$ et $Q_{3e}$ sont des groupes COOH (généralement salifiés par des bases pharmacologiquement acceptables)

$Q_{2e}$ et $Q_{4e}$ ont les significations données pour $Q_{2a}$.

6 - Les composés de formule

14.

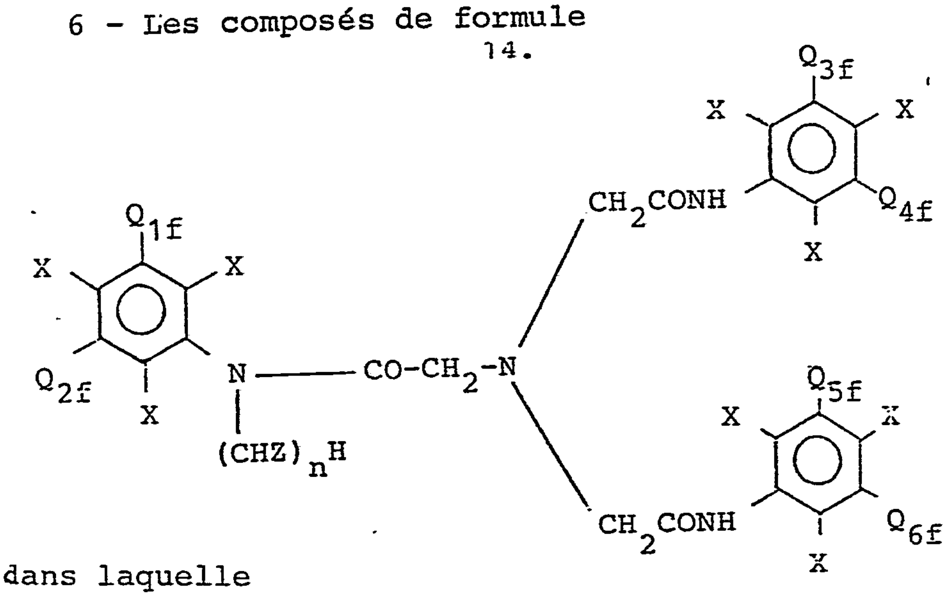

$(IV_a)$

dans laquelle

X est Br ou I

$Q_{1f}$ est un groupe COOH (généralement salifié par une base pharmacologiquement acceptable)

$Q_{2f}$, $Q_{3f}$, $Q_{4f}$, $Q_{5f}$ et $Q_{6f}$ ont les significations données pour $Q_{2a}$, Z est H ou OH et n = 0 à 6.

7 - Les composés de formule

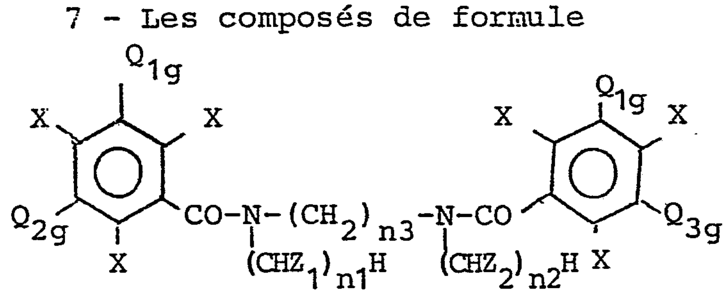

(IIc)

dans laquelle :

X est Br ou I,

$Q_{1g}$ représente un radical de formule

$$-\underset{\displaystyle R'_{27}}{\underset{|}{N}} - COR_{27}$$

$R_{27}$ étant un radical alcoyle (inf.) polyhydroxylé, $R'_{27}$ étant un atome d'hydrogène ou un radical alcoyle inférieur,

$Q_{2g}$ et $Q_{3g}$ représentent indépendamment l'un de l'autre un radical de formule

$- CON\begin{matrix} R_{28} \\ R_{29} \end{matrix}$ , $R_{28}$ et $R_{29}$ étant un atome d'hydrogène, un radical alcoyle inférieur, hydroxyalcoyl inférieur ou alcanoyl (inf.) oxyalcoyle (inf.), un groupe amino ou un radical de formule $-\underset{\displaystyle R_{31}}{\underset{|}{N}} - COR_{30}$

$R_{30}$ étant un radical alcoyle inf., hydroxy alcoyle inférieur ou alcoxy (inf.) alcoyle (inf.) et $R_{31}$ un atome d'hydrogène, un radical alcoyle inférieur ou hydroxyalcoyle inférieur,

$Z_1$ et $Z_2$ sont H ou OH

$n_1$ et $n_2$ = 0 à 6

$n_3$ = 0 à 4.

8. Les composés de formule

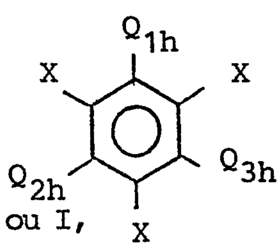

(Id)

dans laquelle X est Br ou I,

$Q_{1h}$, $Q_{2h}$ et $Q_{3h}$ représentent un groupe -COOH, un groupe -COOH salifié par une base pharmacologiquement acceptable ou un groupe de formule $- CO-N\begin{matrix} R_{32} \\ R_{33} \end{matrix}$

dans laquelle $R_{32}$ et $R_{33}$ représentent un atome d'hydrogène, un radical alcoyle inférieur, hydroxyalcoyle (inf.) ou alcanoyl(inf.) oxyalcoyle (inf.).

7. Produits opacifiants selon l'une quelconque des revendications 3 à 6, caractérisés en ce qu'ils sont constitués d'une solution aqueuse d'un mélange de composés iodobenzéniques et de composés bromobenzéniques.

Office européen des brevets

**0074309**
Numéro de la demande

# RAPPORT DE RECHERCHE EUROPEENNE

EP 82 40 1580

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
| --- | --- | --- | --- |
| A | FR-A-2 085 636 (LAB. A.GUERBET) *Revendications* | 1-7 | A 61 K 49/04 |
| A | FR-A-2 074 734 (LAB. A.GUERBET) *Revendications* | 1-7 | |
| A | FR-M- 2 162 (NYEGAARD & CO A/S) *Exemple 2; résumé* | 1-7 | |
| A | FR-M- 820 (NYEGAARD & CO A/S) *Exemple 3, résumé* | 1-7 | |
| A | GB-A-1 033 695 (MALLINCKRODT CHEMICAL WORKS) *Exemple 1* | 1-7 | **DOMAINES TECHNIQUES RECHERCHES (Int. Cl. ³)** |
| A | FR-A-1 088 590 (SCHERING A.G.) *Exemple 5, résumé* | 1-7 | A 61 K 49/00 |
| A | FR-A-2 272 640 (LABS. A.GUERBET) *Exemples 1,18,19; revendications* | 1-7 | |
| D,A | FR-A-2 253 509 (LABS. A.GUERBET) *Exemple 4* | 1-7 | |

Le présent rapport de recherche a été établi pour toutes les revendications

| Lieu de la recherche | Date d'achèvement de la recherche | Examinateur |
| --- | --- | --- |
| LA HAYE | 25-11-1982 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82